# EUROPEAN PATENT SPECIFICATION

(11) **EP 3 784 187 B1**
(45) Date of publication and mention of the grant of the patent: **25.10.2023**
(21) Application number: 18916306.6
(22) Date of filing: 24.04.2018
(51) Int. Cl.: A61F 13/494, A61F 13/537, A61F 13/49, A61F 13/496

(54) **ABSORBENT PANT HAVING AN ABSORBENT CORE WITH CHANNELS**
ABSORBIERENDES HÖSCHEN MIT EINEM ABSORBIERENDEN KERN MIT KANÄLEN
CULOTTE ABSORBANTE AYANT UN NOYAU ABSORBANT AVEC DES CANAUX

(43) Date of publication of application: 03.03.2021
(73) Proprietor: The Procter & Gamble Company, Cincinnati, OH 45202 (US)
(72) Inventor: KREUZER, Carsten, 65824 Schwalbach at Taunus (DE); BIANCHI, Ernesto, 65824 Schwalbach at Taunus (DE); EHRNSPERGER, Bruno Johannes, 65824 Schwalbach at Taunus (DE); STOELZEL, Claus Peter, 65824 Schwalbach at Taunus (DE); TONG, Ling, Beijing 101312 (CN)
(74) Representative: P&G Patent Germany
(86) International application number: PCT/CN2018/084173
(87) International publication number: WO 2019/204973

(56) References cited:
- WO-A1-2015/031225
- CN-A- 104 837 453
- CN-A- 104 853 704
- CN-A- 105 530 902
- CN-A- 105 579 008
- US-A1- 2016 206 482
- US-A1- 2017 312 147
- US-A1- 2018 021 187

## Description

### BACKGROUND OF THE INVENTION

Absorbent pants are well known and widely used, both for babies and infants as well as for adult incontinence. A particular type of absorbent pant design currently marketed is sometimes called the "balloon" pant. The balloon pant design usually includes a central absorbent chassis, including the absorbent core, and an elastic belt. The elastic belt is usually relatively wide (in the longitudinal direction) and elastically stretchable in the lateral direction. It entirely encircles the wearer's waist. The belt is often formed of two layers of nonwoven web sandwiching one or more elastic members, such as a plurality of laterally-oriented strands or strips of elastomeric material, or a section of elastomeric film, elastomeric scrim or elastomeric nonwoven. It is common among such designs that, in manufacture, the elastic member(s) are sandwiched between the nonwoven web layers while in a strained condition.

The absorbent core that is part of the central chassis plays an important role in containment and absorbency of exudates, as well as in comfort, fit and appearance of the pant when worn. In recent years, absorbent core designs for both absorbent pants and taped absorbent diapers have progressed toward structures with relatively higher proportions by weight of superabsorbent polymer particles and lower proportions of cellulose fibers (which are sometimes also referred to as "pulp" or "airfelt"), resulting in structures that are thinner than absorbent core designs of earlier years and enabling manufacture of products that are less bulky and more closely-fitting (e.g., more underwear-like) when dry. The latter absorbent core designs, however, can be slower in liquid acquisition rate, and because of their greater proportions of absorbent polymer particles, can become saggy, bulky and gelatinous when wetted. To address these issues, absorbent cores including longitudinally-oriented channels have been developed. Appropriately located and structured longitudinal channels can help distribute liquid along deposits of absorbent polymer particles along the length of the absorbent core, and thereby help improve acquisition rate. They also can help reduce the likelihood of a saggy and bulky appearance of the article when wetted, by providing longitudinal structural rigidity through the crotch region of the article resulting from pressure within the wetted absorbent polymer particle deposits between the channels.

It has now been found, however, that absorbent cores provided with longitudinally extending channels which work well for taped diapers, can provide certain disadvantages when being used in absorbent pants. Hence, there remains to be room for improvement regarding absorbent core designs having channels which are especially beneficial for use in absorbent pants.

US 2018/021187 relates to a disposable absorbent pant having a belt structure that encircles the wearer's waist, with front and rear belt portions, and a longitudinally channeled absorbent core structure. The channels are extended so as to underlie at least one of the front and rear belt portions.

US 2016/206482 discloses a disposable absorbent pant with a belt structure and a longitudinally channeled absorbent core structure. The channels include at least one shorter, at least partially transversely-oriented secondary channel in the front and/or rear.

WO 2015/031225 is concerned with an absorbent article comprising a liquid management system (LMS) and an absorbent core disposed at least partially intermediate a topsheet and a backsheet. The LMS defines one or more channels therein. The one or more channels of the LMS may at least partially overlap or not overlap with channels defined in the absorbent core.

### SUMMARY OF THE INVENTION

Upon investigation, the inventors have found that one of the disadvantages that can arise with absorbent cores having longitudinally extending channels is closely associated with the difference in the way a taped diaper is applied onto a wearer versus the way in which an absorbent pant is applied. A taped diaper is normally applied by laying the wearer on the inner surface of the rear waist region of the diaper and pulling the front waist region up over the wearer's belly such that the crotch region of the absorbent article is placed between the wearer's legs. In contrast, an absorbent pant is typically applied by having the wearer's feed and legs sliding through the leg openings of the pant and pulling the pant upwards such that the front and rear waist regions of the pant lie against the belly and the back of the wearer with the crotch portion of the pant positioned between the legs of the wearer.

If the absorbent core has longitudinally extending channels, the absorbent article tends to fold along these channels. Due to pulling the absorbent pant upwards between the wearer's legs, the absorbent core is "squeezed" between the legs of the wearer while being pulled. As a consequence, an absorbent core having longitudinally extending channels typically forms a U-shape with the edges of the absorbent core adjacent to the legs of the wearer being folded upwardly and, to some extend also somewhat inwardly. Due to the U-shape, the absorbent core may not fit optimally between the legs of the wearer, providing reduced surface which is in close contact with the body of the wearer in the crotch. The folding tends to continue to the regions outside the crotch and towards the front and rear waist region, leading to a narrowed absorbent core in these regions as well and reduced contact of the absorbent core with the body of the wearer. This may increase the risk of leakage during use. It also leads to reduced body surface coverage especially over the buttocks of the wearer.

The present invention thus provides an absorbent core having an improved channel configuration which is especially suitable for pant-type absorbent articles.

The invention relates to a disposable absorbent pant, having a longitudinal axis, a lateral axis, a front waist region, a rear waist region and a crotch region between the front and rear waist regions.

The pant comprises a central chassis extending from the front waist region through the crotch region to the rear waist region. The central chassis comprises a topsheet, a backsheet and an absorbent core placed in between the topsheet and the backsheet, the central chassis having a laterally extending chassis front edge, a laterally extending chassis rear edge and first and second longitudinally extending chassis side edges.

An elasticized front belt is provided in the front waist region and an elasticized rear belt provided in the rear waist region, the front and rear belt each having a body-facing surface and a garment-facing surface. The front belt having a transversally extending front waist edge, the rear belt having a transversally extending rear waist edge, the front and rear belt each having a first and second longitudinally extending side edge, with the first side edge of the front belt being joined to the first side edge of the rear belt and the second side edge of the front belt being joined to the second side edge of the rear belt at side seams to form a waist opening and two leg openings.

The absorbent core comprises an absorbent layer, the absorbent layer having a laterally extending front edge, a laterally extending rear edge and first and second longitudinally extending side edges. The absorbent layer comprises a first and a second longitudinally-oriented elongate channel formed therein.

The first channel is provided between the longitudinal axis and the first side edge of the absorbent layer, the second channel is provided between the longitudinal axis and the second side edge of the absorbent layer.

The first and second channel each have a channel front end towards the absorbent layer's front edge, a channel rear end towards the absorbent layer's rear edge and a center which is equally spaced from the channel's front and rear ends across the longitudinal axis, each of the first and second channel being curved such that the first and second channel are closer to the longitudinal axis at a location (herein after referred to as "necking point") between the front end and the rear end of the respective first and second channel than the channel's front and rear ends.

The necking point may be located between the center and the rear end of the respective first and second channel. The necking point of the first channel may be spaced from 5% to 30%, or from 5% to 25%, or from 10% to 25% away from the center of the first channel towards the rear end, based on the total length of the first channel, as measured along a straight line from the front end to the rear end of the first channel.

Likewise, the necking point of the second channel may be spaced from 5% to 30%, or from 5% to 25%, or from 10% to 25% away from the center of the second channel towards the rear end, based on the total length of the second channel, as measured along a straight line from the front end to the rear end of the second channel.

The first and second channel may not be closer to the longitudinal axis at any other location than at their necking point.

The first channel may follow a first curved path with only one curve between the necking point and the rear end of the first channel. The first channel may follow a second curved path with only one curve between the necking point and the front end of the first channel. The first curved path of the first channel may have a steeper curvature compared to the second curved path of the first channel. The first and the second curved path of each of the first and second channel are concavely shaped relative to the longitudinal axis. This is also reflected in the Figures.

Likewise, the second channel may follow a first curved path with only one curve between the necking point and the rear end of the second channel. The second channel may follow a second curved path with only one curve between the necking point and the front end of the second channel. The first curved path of the second channel may have a steeper curvature compared to the second curved path of the second channel.

The first and second channel are spaced apart from each other at their necking point by a first distance L1. the absorbent layer has a first transverse width W1 measured from the first longitudinally extending side edge to the second longitudinally extending side edge of the absorbent layer across the necking point of the first and second channel. The distance L1 is from 30 mm to 50 mm, or from 32 mm to 45 mm, or from 35 mm to 45 mm.

The first and second channel are spaced apart from each other at their rear ends by a second distance L2. The absorbent layer has a second transverse width W2 measured from the first longitudinally extending side edge to the second longitudinally extending side edge of the absorbent layer across the rear ends of the first and second channel. The ratio of W2 to L2 is from 1.5 to 2.8, or from 1.8 to 2.5.

The ratio of W1 to L1 is higher than the ratio of W2 to L2.

The ratio of L2 to L1 is from 1.2 to 2.5, or from 1.4 to 2.2, or from 1.5 to 2.0.

The first and second channel may be spaced apart from each other at their front ends by a third distance L3. The absorbent layer has a third transverse width W3 measured from the first longitudinally extending side edge to the second longitudinally extending side edge of the absorbent layer across the front ends of the first and second channel. The ratio of W3 to L3 may be from 1.5 to 2.8, or from 1.8 to 2.5.

The ratio of W 1 to L1 may be higher than the ratio of W3 to L3.

The ratio of L3 to L1 may be from 1.2 to 2.5, or from 1.4 to 2.2, or from 1.5 to 2.0.

The ratio of W1 to L1 may be from 2.5 to 4.5, or from 2.8 to 4.0, or from 3.0 to 3.8.

L2 may be the same as L3. Still alternatively, L2 and L3 may not differ from each other by more than 30%, or not more than 20%, or not more than 10%, or not more than 5% based on the longer distance.

The absorbent layer of the pant may have no channel other than the first and second channel. The absorbent layer may also have no other areas, i.e. areas which are not in the shape of a channel, which are substantially free or free of absorbent material apart from the first and second channel.

Each of the front and rear belt of the pant may comprise or may be formed of an inner layer, an outer layer and a plurality of elastic members disposed between the inner layer and the outer layer. The transversally lower edges of the front and the rear belt may be formed by one or both of the inner and outer layers.

The absorbent core of the pant may comprise a first substrate layer, such as a nonwoven web, provided towards the backsheet, and a second substrate layer, such as a nonwoven web, provided towards the topsheet, and absorbent material provided between first and second substrate layer, wherein the absorbent material forms the absorbent layer.

The first and second channel in the absorbent core may each be substantially free, or free of absorbent material and the first and second substrate layer may be directly bonded to each other in the first and second channels.

The front end of each of the first and second channel may overlap with the front belt.

The central chassis may be attached to the front belt and to the rear belt. The attachment may be done by any means known in the art. For example, the central chassis may be attached to the front belt and to the rear belt by adhesive bonding, ultrasonic bonding, thermal bonding, pressure bonding, or combinations thereof. The central chassis may be attached to the front belt at least along an area at and/or adjacent to the central chassis front edge. The central chassis may be attached to the rear belt at least along an area at and/or adjacent the central chassis rear edge. The central chassis may be attached to the front and rear belt by attaching the garment-facing surface of the central chassis to the body-facing surface of the front and rear belt.

The central chassis may be provided in the disposable absorbent pant such that the chassis front edge is longitudinally spaced from the front waist edge of the pant and/or the chassis rear edge is longitudinally spaced from the rear waist edge (i.e. the chassis front edge is closer to the lateral axis that the front waist edge and/or the chassis rear edge is closer to the lateral axis than the rear waist edge). The spacing between the front waist edge and the chassis front edge may be smaller than the spacing between the rear waist edge and the chassis rear edge. E.g. the spacing between the front waist edge and the chassis front edge may be from 1.1 to 3 times, or from 1.5 to 3 times longer than the spacing between rear waist edge and the chassis rear edge.

### BRIEF DESCRIPTION OF THE DRAWINGS

In the drawings, the same features are numbered consistently throughout the various views and depictions of examples.
Fig. 1 is a front perspective view of an example of a disposable absorbent pant of the present invention.
Fig. 2A is a schematic plan view of a disposable absorbent pant of the present invention prior to joining of the front and rear belt at side seams, body-facing surfaces facing the viewer.
Fig. 2B is a schematic plan view of a disposable absorbent pant of the present invention prior to joining of the front and rear belt at side seams, body-facing surfaces facing the viewer.
Fig. 2C is a schematic plan view of a disposable absorbent pant of the present invention prior to joining of the front and rear belt at side seams, body-facing surfaces facing the viewer.
Fig. 3 is a schematic, exploded perspective view of components of a belt portion.
Fig. 4 is a schematic, close-up plan view of an area of a belt portion.
Fig. 5 is a schematic cross section of the area of the belt portion shown in Fig. 4.
Fig. 6A is a schematic side view of a disposable absorbent pant of the present invention.
Fig. 6B is a schematic side view of a disposable absorbent pant of the present invention.
Fig. 7 is a schematic perspective view of an absorbent layer with a first substrate layer and including a first and second channel for use in a disposable absorbent pant of the present invention.
Fig. 8 is a schematic perspective view of an absorbent layer including a first and second channel for use in a disposable absorbent pant of the present invention.
Fig. 9A is a schematic top/plan view of an absorbent layer with a first substrate layer and including a first and second channel for use in a disposable absorbent pant of the present invention.
Fig. 9B is similar to Fig. 9A and additionally shows (in dotted lines) fold lines continuing outwardly from the first and second channel towards the rear edge of the absorbent layer.
Fig. 10A is a schematic lateral cross-section view of a central chassis through the first and second channel.
Fig. 10B is a schematic lateral cross-section view of a main chassis outside the first and second channel.
Fig. 11A is a schematic plan view of a disposable absorbent pant of the present invention, prior to joining of the front and rear belt at side seams, wearer- facing surfaces facing the viewer, shown with first and second channel in dotted lines.
Fig. 11B is a schematic plan view of an alternate disposable absorbent pant of the present invention, prior to joining of the front and rear belt at side seams, body-facing surfaces facing the viewer, shown with first and second channel in dotted lines.
Fig. 11C is a schematic, exploded longitudinal cross section view of a portion of the disposable absorbent pant depicted in Fig. 11A.
Fig. 12 is a schematic plan view of a disposable absorbent pant of the present invention prior to joining of the front and rear belt at side seams, garment-facing surfaces facing the viewer, shown with non-elasticized zones in front and rear belt.

### DETAILED DESCRIPTION OF THE INVENTION

As used herein, "absorbent article" refers to a device that absorbs and contains body exudates, and, more specifically, refers to a device that is placed against or in proximity to the body of the wearer to absorb and contain the various exudates discharged from the body. Absorbent articles include diapers (baby diapers and diapers for adult incontinence), pants (for babies, infants and/or for adults), absorbent inserts (which are intended to be inserted into an outer cover to form a diaper or pant), feminine care absorbent articles such as sanitary napkins or pantiliners, and the like. As used herein, the term "exudates" includes, but is not limited to, urine, blood, vaginal discharges, sweat and fecal matter. Preferred absorbent articles of the present invention are disposable absorbent articles, more preferably disposable diapers and disposable absorbent pants.

As used herein, "disposable" is used in its ordinary sense to mean an article that is disposed or discarded after a limited number of usage over varying lengths of time, for example, less than 20 usages, less than 10 usages, less than 5 usages, or less than 2 usages. If the disposable absorbent article is a diaper, a pant, sanitary napkin, sanitary pad or wet wipe for personal hygiene use, the disposable absorbent article is most often intended to be disposed after single use. The absorbent articles described herein are disposable.

As used herein, the term "comprises" is an open-ended term which means that other features, components, items or steps can be added. The term "comprises" as used herein includes the terms "essentially consisting of" and "consist of". "Consist of" denotes that only the features, components or steps following the term "consist of" are included with no further features, components, items or steps.

As used herein, "diaper" and "pant" (herein also referred to as "absorbent pant") refers to an absorbent article generally worn by babies, infants and incontinent persons about the lower torso so as to encircle the waist and legs of the wearer and that is specifically adapted to receive and contain urinary and fecal waste. In a pant, as used herein, the longitudinal edges of the first and second waist region are attached to each other to a pre-formed waist opening and leg openings. A pant is placed in position on the wearer by inserting the wearer's legs into the leg openings and sliding the pant absorbent article into position about the wearer's lower torso. A pant may be pre-formed by any suitable technique including, but not limited to, joining together portions of the absorbent article using refastenable and/or non-refastenable bonds (i.e. with permanent side seams not intended to be torn upon prior to removal of the pant from the wearer for disposal). In a diaper, the waist opening and leg openings are only formed when the diaper is applied onto a wearer by (releasable) attaching the longitudinal edges of the first and second waist region to each other on both sides by a suitable fastening system.

"Longitudinal" means a direction running substantially perpendicular from a waist edge to a longitudinally opposing waist edge of an absorbent article when the article is in a flat out, uncontracted state.

"Transverse" and "lateral" are used interchangeable herein and both terms refer to a direction running from a longitudinally extending side edge to a transversally opposing longitudinally extending side edge of an absorbent article and generally at a right angle to the longitudinal direction.

"Body-facing" and "garment-facing" refer respectively to the relative location of an element or a surface of an element or group of elements. "Body-facing" implies the element or surface is nearer to the wearer during wear than some other element or surface. "Garment-facing" implies the element or surface is more remote from the wearer during wear than some other element or surface (i.e., element or surface is proximate to the wearer's garments that may be worn over the disposable absorbent article).

"Hot melt adhesive" as used herein refers to adhesives conforming with the description given in "Adhesion and Adhesives Technology: An Introduction" by Alphonsus V. Pocius (Hanser publishers Munich, 1997). Therein a hot melt is defined as an adhesive applied from the melt and gaining strength upon solidification.

A "nonwoven" is a manufactured sheet or web of directionally or randomly oriented fibers which are first deposited and accumulated onto a moving surface (such as a conveyor belt) and then consolidated and bonded together by friction, cohesion, adhesion or one or more patterns of bonds and bond impressions created through localized compression and/or application of pressure, heat, ultrasonic or heating energy, or a combination thereof. The term does not include fabrics which are woven, knitted, or stitch-bonded with yarns or filaments. The fibers may be of natural and/or man-made origin and may be staple and/or continuous filaments or be formed in situ. Commercially available fibers have diameters ranging from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). Nonwovens may be formed by many processes including but not limited to meltblowing, spunbonding, spunmelting, solvent spinning, electrospinning, carding, film fibrillation, melt-film fibrillation, air laying, dry-laying, wet laying with staple fibers, and combinations of these processes as known in the art. The basis weight of a nonwoven is usually expressed in grams per square meter (g/m²).

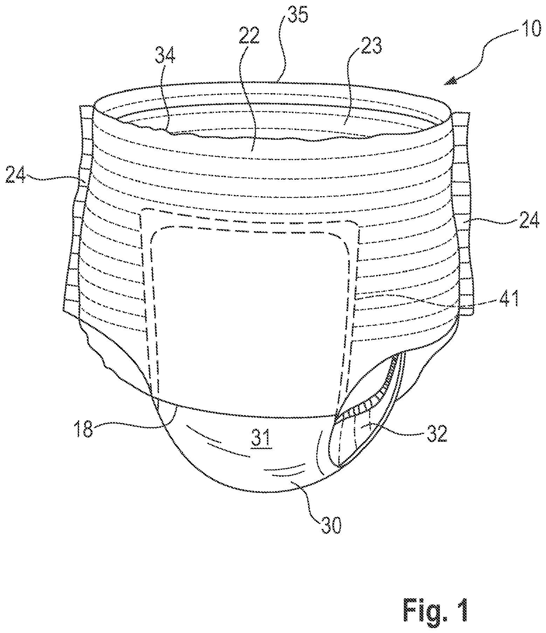

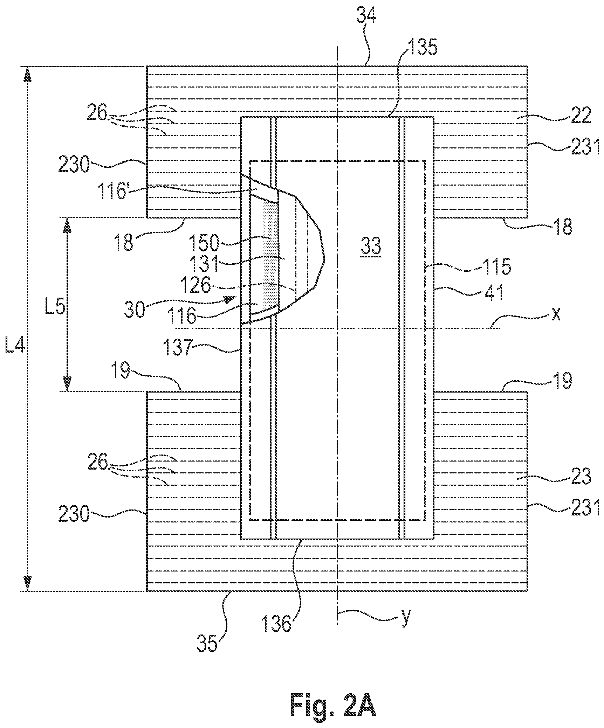

Fig. 1. depicts an example of a balloon-type absorbent pant 10. Figs. 2A-2C depict examples of a pant in an open configuration laid out flat and stretched out laterally against elastic - induced contraction, body-facing surfaces facing the viewer, prior to final assembly in which front belt portion 22 is joined to rear belt portion 23 at seams 24. To form pant 10, the article may be folded at or about lateral axis x (located at the longitudinal midpoint of the article) with the topsheet 33 facing inward, and the longitudinal edges of the front 22 and rear 23 belt portions joined at seams 24, forming a pant structure having leg openings 15, front waist edge 34 and rear waist edge 35.

The pant structure includes an elasticized front belt 22, an elasticized rear belt 23 and a central chassis 30. Central chassis 30 may include any combination of components found in the absorbent structures of disposable diapers and absorbent pants. The central chassis 30 comprises a liquid impermeable backsheet 31, a liquid permeable topsheet 33, an absorbent core positioned between the topsheet and the backsheet. The central chassis 30 may further comprise elasticized barrier cuffs 32. Examples and descriptions of components and configurations of a central chassis 30 may be found in U.S. Pat. App. Pub. No. 2013/0211355, as well as in the other references cited herein, to the extent not inconsistent herewith, wherein the chassis described includes components and features that may be included in central chassis 30. In the example shown in Fig. 1, the front belt 22 stops at lower edge 18, thus delimiting the front waist region against the crotch region. Central chassis 30 may overly front and rear belts 22, 23 on the inside (body-facing surface) thereof. The outer perimeter 41 of the central chassis 30 may be defined by the outer perimeter of the backsheet 31.

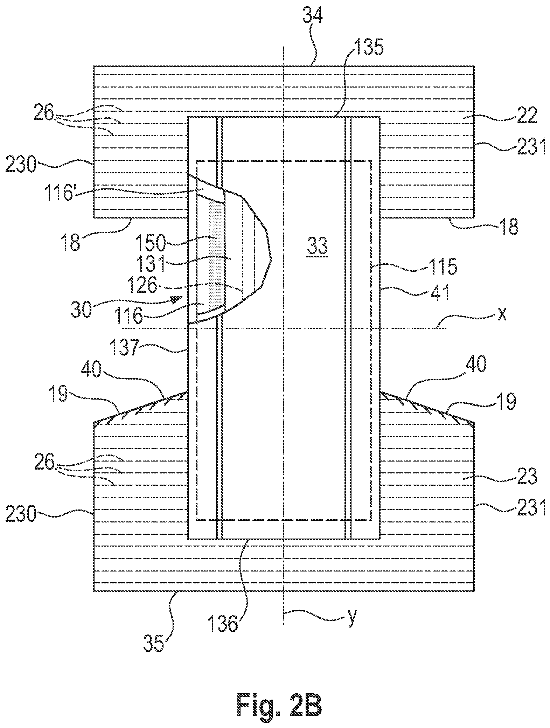

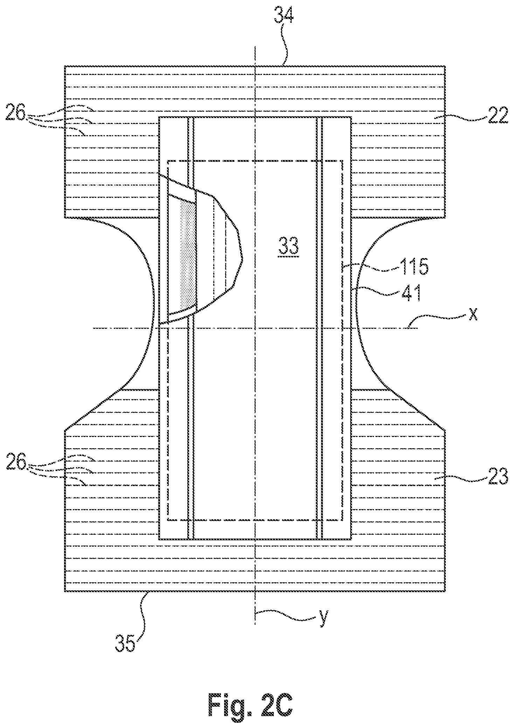

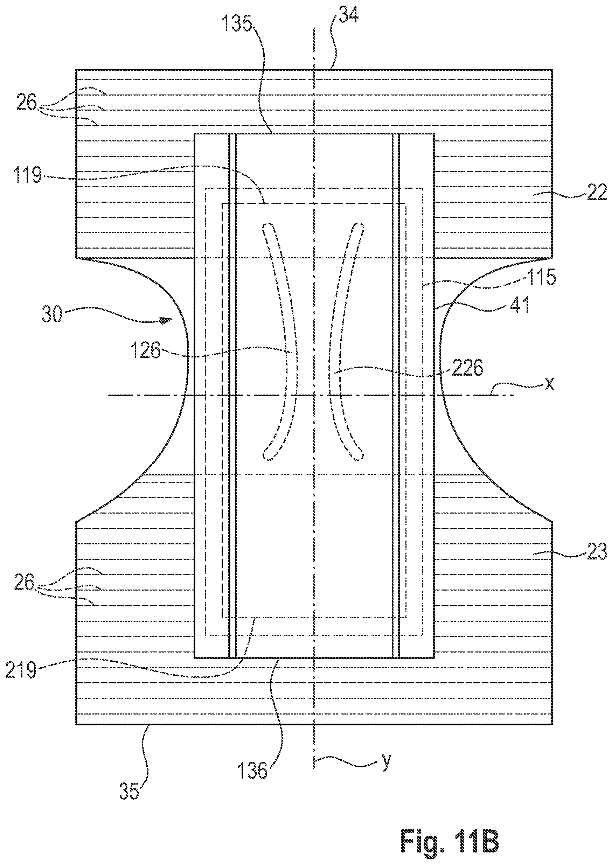

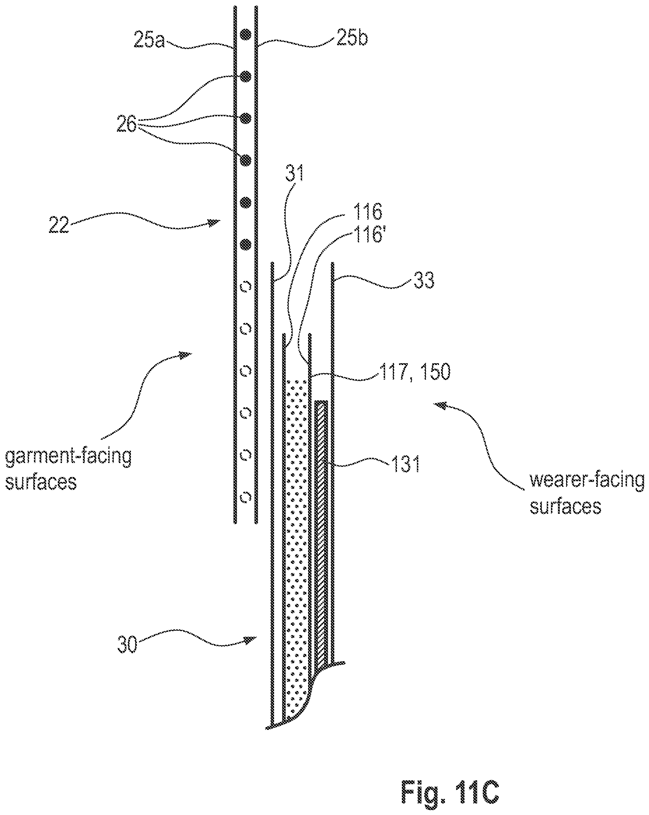

In the examples shown in Figs. 2A and 2B, front and rear belt 22, 23 may be the outermost structures forming the front and rear waist regions of the pant. In the example illustrated in Fig. 2C (also as shown in Fig. 11B), the front and rear belt 22, 23 of the pant may extend into the crotch region such that one or more layers forming the front belt and the rear belt are continuously extending from the front waist edge 34 to the rear waist edge 35. In such pant configurations, the front belt and the rear belt are notionally divided at the transverse axis x of the pant. At least one layer may extend from the front waist edge to the rear waist edge and be formed of a continuous nonwoven web. One or more additional layers may not extend over the complete longitudinal dimension of the pant. As reflected in Fig. 2C, the one or more layers extending from the front waist edge to the rear waist edge may be cut to a profile providing suitably tailored leg opening edge profiles as desired.

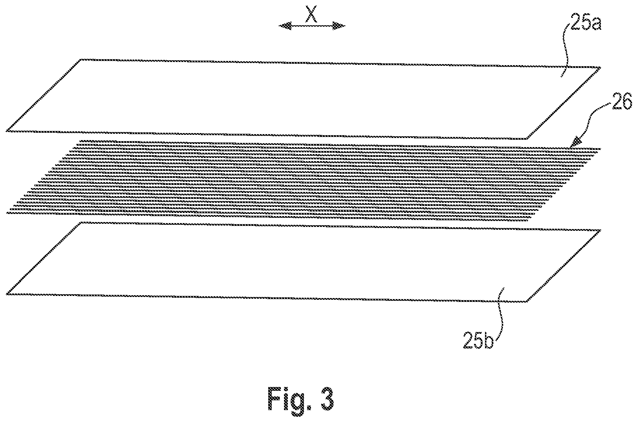

Referring to Figs. 3-5, one or both of front and rear belt 22, 23 may be formed of layers of nonwoven web 25a, 25b, which respectively form inner and outer layers. Suitable nonwoven web materials that may be useful in the present invention also include, but are not limited to spunbond, spunlaid, meltblown, spunmelt, solvent-spun, electrospun, carded, film fibrillated, melt- film fibrillated, air-laid, dry-laid, wet-laid staple fibers, and other nonwoven web materials formed in part or in whole of polymer fibers, as known in the art. The nonwoven web may be formed predominately of polymeric fibers. Suitable non- woven fiber materials may include, but are not limited to polymeric materials such as polyolefins, polyesters, polyamide, or specifically, polypropylene (PP), polyethylene (PE), poly-lactic acid (PLA), polyethylene terephthalate (PET) and/or blends thereof.

For purposes herein, use of a nonwoven web formed of crimped bicomponent or multicomponent fibers may be desired as one both layers 25a, 25b used to form the belt portions, because they can feel particularly soft to the touch (for wearer comfort on the inside and aesthetically pleasing feel on the outside) and are generally quite pliable.

Referring to Figs. 3-5, layers of nonwoven web 25a, 25b comprised by the front and rear belt 22, 23 may sandwich one or more elastic members such as a plurality of elastic strands 26. Elastic strands may be formed of an elastomeric material, such as an elastane (for example, LYCRA HYFIT fiber, a product of Invista, Wichita, Kansas). Layers of nonwoven web 25a, 25b may be joined together about elastic strands 26 by adhesive deposited between the layers, by thermal bonds, by compression bonds, or by a combination thereof. In other examples, the one or more elastic members may be strips or a section of film formed of elastomeric material. Where the elastic member is elongate, it may be desirable that the longer dimension be laterally oriented, or even substantially aligned with the lateral direction, as strands 26 are depicted in the figures.

The elastic members can also be formed from various other materials, such as but not limited to, rubbers, styrene ethylbutylene styrene, styrene ethylene propylene styrene, styrene ethylene ethylene propylene styrene, styrene butadiene styrene, styrene isoprene styrene, polyolefin elastomers, elastomeric polyurethanes, and other elastomeric materials known in the art, and combinations thereof. The elastic members may be extruded strand elastics with any number of strands (or filaments). The elastic members can have a decitex ranging from 50 to 2000. The elastic members may be in a form of film. Examples of films have been described in prior patent applications (see, for example, U.S. Pat. App. Pub. No. 2010/0040826).

Still referring to Figs. 3-5, during manufacture of the front and rear belt 22, 23, the elastic members such as elastic strands 26 may be pre-strained lengthwise by a desired amount as they are being incorporated into the front and rear belt 22, 23 between layers of nonwoven web 25a, 25b. Upon subsequent relaxation of the belt, the elastic members, such as elastic strands 26, will contract laterally toward their unstrained lengths. This causes the layers of nonwoven web 25a, 25b to gather and form ruffles or rugosities 27 having ridges 28 and valleys 29 generally transverse to the lengths of the elastic strands 26, and extending in the z-direction (i.e. the direction perpendicular to both the lateral and the longitudinal axis).

In another example, to adhere the components of the belt laminate, the elastic strands 26 themselves may be individually coated with adhesive ("strand coated") prior to incorporation between layers of nonwoven web 25a, 25b to form the front and rear belt 22, 23. Various coating methods and techniques, including strand coating methods and techniques, are shown for example in U.S. Pat. Nos. 5,340,648; 5,501,756; 5,507,909; 6,077,375; 6,200,635; 6,235,137; 6,361,634; 6,561,430; 6,520,237; 6,582,518; 6,610,161; 6,613,146, 6,652,693, 6,719,846 and 6,737,102. The adhesive used may be a hot-melt type adhesive having elasticity and flexibility making it suitable for attaching pre-strained elastic materials to substrates, such as OMNIMELT BLOCKS 22 H2401F, or ZEROCREEP brands such as A VANCE, available from Bostik, Inc., Wauwatosa, Wisconsin.

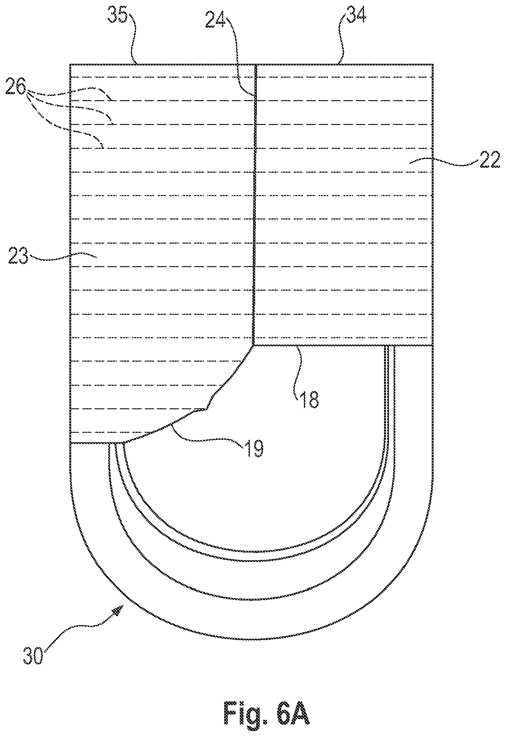

Referring e.g. to Fig. 2A, the rear belt 23 may have a greater longitudinal dimension (i.e., greater length) than the front belt 22. This may help provide greater coverage of the wearer's buttocks area in the rear while providing greater comfort in front, via better conformity with wearer anatomy and natural body movement. In the example of Fig. 2A, when the front and rear belt 22, 23 are joined at side seams 24 along their respective first and second longitudinally extending side edges at side seams with their respective waist edges 34, 35 substantially aligned, however, the second lower edge 19 of the rear belt 23 will lie below the first lower edge 18 of the front belt 22 to form a stepped leg edge profile at the seams 24. If deemed undesirable, this effect may be mitigated by selecting, disposing and/or varying pre-strain levels among the elastic members as suggested and described in, for example, U.S. Pat. App. Ser. No. 62/042387, to laterally draw the lower rear corners of the rear belt 23 (i.e. the corner where the first and second longitudinally extending side edges of the rear belt meets the second lower edge 19 of the rear belt 23) inward toward the longitudinal axis y. A potential desirable result of such practice is schematically suggested in Fig. 6A.

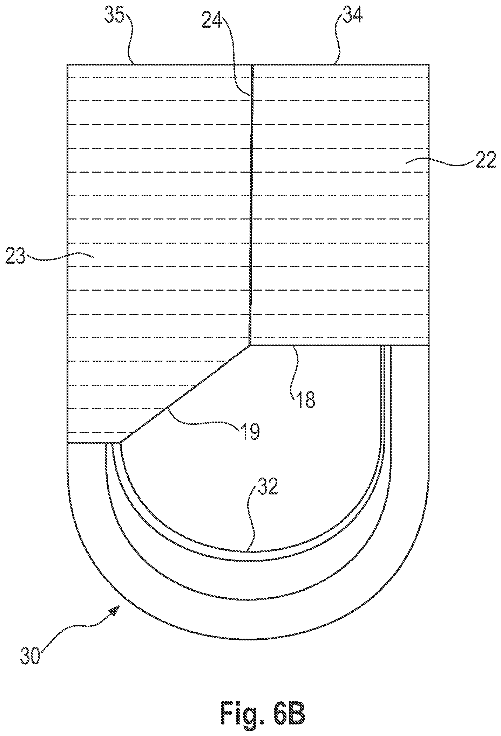

Alternatively, the lower portions of the first and second longitudinally extending side edges below the side seams 24 of the rear belt 23 and/or the second lower edges 19 laterally outside of the area where the rear belt 23 overlays the central chassis 30 may be trimmed off as suggested in Figs. 2B and 6B. Such trimming may be done along straight lines as suggested in Figs. 2B and 6B, or may follow a trim path that are curved (as exemplified in Fig. 6A) and either concave or convex with respect to the remaining area of the rear belt 23, as may be desired to impart a particular curved rear leg edge profile.

In conjunction with such trimming and the configuration of elastic strands described above, it may be desired to impart bonding 40 between layers 25 a, 25b along the trimmed second lower edges 19 and/or the lower portions of the first and second longitudinally extending side edges below the side seams 24 of rear belt 23. Such bonding may serve to prevent any separation of the layers along the edges that may contribute to creating a ragged appearance, and may also help the rear belt 23 more effectively draw inward laterally toward the central chassis 30, under the contractive force of the elastic strands below the side seams 24.

Bonding 40 may be effected by mechanical/compression bonds as described in, for example, U.S. Pats. Nos. 4,854,984 and 4,919,738, by thermal bonds or welds, or by deposits of adhesive between nonwoven layers 25 a, 25b. As suggested in Fig. 2B, such bonding may form a pattern along the edges. Such bonding may be supplemental to any bonding between layers 25a, 25b generally holding rear belt 23 together as a laminate structure.

Side seams 24 may be permanent or refastenable. Permanent seams may be formed between the front belt portion and the rear belt portion by any bonding mechanism wherein the first and second longitudinally side edges 230, 231 of front and rear belt 22, 23 may not be forcibly separated without substantial damage to one or both of the front and rear belt 22, 23. Bonding forming permanent side seams 24 may include pressure bonding, thermal bonding/welds, ultrasonic bonding, adhesive bonding or combinations thereof.

Refastenable side seams 24 may be formed between the front belt 22 and the rear belt 23 by any mechanism configured to permit substantially non-destructive forcible separation of the front and rear belt 22, 23, and subsequent substantial reattachment or refastening at the same locations. One example of such mechanism is a hook-and-loop fastening system, for example, a VELCRO fastening system. A suitably sized and shaped hook component may be bonded to one of the front or rear belt along the first and second longitudinal side edges 230, 231 thereof, and suitably sized and shaped loops component may be bonded to the other of the front or rear belt along the first and second longitudinal edges 230, 231 thereof, in positions in which they may be brought together and engaged to form seams 24. Examples are depicted in U.S. Pat. App. Serial Nos. 61/787,416; 61/787,332; 61/666,065.

### Absorbent Core

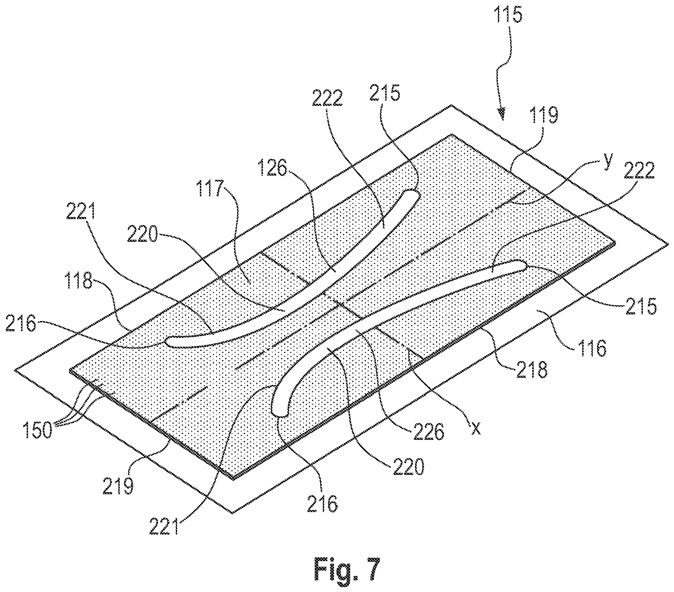

The absorbent core 115 includes an absorbent layer 117 which may include superabsorbent polymer particles, and optionally cellulose fibers. The absorbent layer 117 may be supported by, and immobilized on, one or more substrate layers 116, 116', such as a first substrate layer 116 provided towards the backsheet 31 of the central chassis 30 and a second substrate layer 116' provided towards the topsheet 33 of the central chassis, with the absorbent layer being sandwiched in between the first and second substrate layer 116, 116'. Examples of absorbent structures 115 are illustrated in Figs. 7, 9, 10A and 10B.

The first and/or second substrate layer 116, 116' of the absorbent core may be any material capable of supporting the superabsorbent polymer particles. It may be a web or sheet material, such as foam, film, woven or, preferably, a nonwoven web. The first and second substrate layer 116, 116' may be distinct separate sheets of material (such as two nonwoven webs) or may be formed of a continuous sheet (such as a continuous nonwoven web) which is wrapped around the absorbent layer.

The first and second substrate layers 116, 116' and the absorbent layer 117 may be coextensive or the first and/or second substrate layer 116, 116' may be slightly longer and wider than the absorbent layer 117 (as suggested in Figs. 7, 9, 10A and 10B).

The absorbent layer 117 may include superabsorbent polymer particles 150, and optionally cellulose fibers. The absorbent layer may include absorbent polymer in other forms such as superabsorbent polymer fibers. Superabsorbent polymer particles. The absorbent layer may include superabsorbent polymer particles combined with cellulose. "Cellulose" as used herein refers to comminuted wood pulp in the form of fibers, sometimes also referred in the art as "air-felt".

The absorbent layer may comprise more than 70%, or more than 80%, or more than 90%, or more than 95% or even 100% of superabsorbent polymer particles by weight of the absorbent layer. The absorbent layer may include superabsorbent polymer particles and less than 5% by weight of cellulose, or less than 2% by weight of cellulose, or even no cellulose. When the absorbent layer is cellulose free, the only absorbent material in the absorbent layer may be superabsorbent polymer (particles or fibers). The resulting absorbent cores have a reduced thickness in the dry state compared to conventional absorbent cores including cellulosic fibers. The reduced thickness helps to improve the fit and comfort of the absorbent article for the wearer.

Alternatively, the absorbent layer may comprise a mixture of superabsorbent polymer particles and cellulose fibers. The absorbent layer may comprise more than 20%, or more than 30%, or more than 40%, by weight of the absorbent layer, of cellulose fibers. The superabsorbent polymer particles and the cellulose fibers may be homogeneously mixed with each other such that the ratio of cellulose fibers to superabsorbent polymer particles is substantially the same throughout the absorbent layer. Alternatively, the superabsorbent polymer particles and the cellulose fibers may be non-homogeneously mixed such that the ratio of cellulose fibers to superabsorbent polymer particles is higher towards the front and rear edges of the absorbent layer compared to a central area of the absorbent layer. The area towards the front edge of the absorbent layer, the area towards the rear edge of the absorbent layer, and the central area may each extend along 113 of longitudinal dimension of the absorbent layer along the longitudinal axis.

### First and second channel

The absorbent layer 117 includes at least a first and a second channel 126, 226. "Channels" as used herein refers to troughs or other identifiable elongate passageways through the deposit of absorbent material in the absorbent layer (see e.g. Figs. 2A, 2B and 7-9).

The channels may extend partially or, preferably, entirely through the thickness of the absorbent layer 117. If the first and second channel 126, 226 extend entirely through the thickness of the absorbent layer 117, substantially no absorbent material is present in the first and second channel 126, 226. "Substantially no absorbent material" means that either no absorbent material at all or insignificant amounts of absorbent material are present. For example, trace amounts of superabsorbent polymer particles and/or cellulose fibers may be present in the first and second channels which may be due to slight deviations in the manufacturing of the absorbent core, which typically runs at high speed.

The first and second channel 126, 226 formed in the absorbent layer 117 are each longitudinally-oriented elongate channels.

The first channel is provided between the longitudinal axis y and the first side edge 118 of the absorbent layer 117. The second channel is provided between the longitudinal axis y and the second side edge 218 of the absorbent layer 117.

The first and second channel 126, 226 each have a channel front end 215 towards the absorbent layer's front edge 119, a channel rear end 216 towards the absorbent layer's rear edge 219 and a center 214 which is equally spaced from the respective channel's front and rear end 215,216 across the longitudinal axis y. Each of the first and second channel 126, 226 is curved such that the first and second channel are closer to the longitudinal axis y at a location (the "necking point" 220) between the front end and the rear end of the respective first and second channel, than the channel's front and rear ends 215, 216.

The necking point may be located between the center and the rear end of the respective first and second channel. The necking point 220 of the first channel 126 may be spaced from 5% to 30%, or from 5% to 25%, or from 10% to 25% away from the center 214 of the first channel towards the rear end 216, based on the total length of the first channel 126, as measured along a straight line from the front end 215 to the rear end 216 of the first channel 126.

Likewise, the necking point 220 of the second channel 226 may be spaced from 5% to 30%, or from 5% to 25%, or from 10% to 25% away from the center 214 of the second channel 226 towards the rear end 216, based on the total length of the second channel 226, as measured along a straight line from the front end 215 to the rear end 216 of the second channel 226.

The first and second channel 126 and 226 may each not be closer to the longitudinal axis y at any other location than at their necking point 220. If the necking point of the first channel 126 is located at a straight section (i.e. a non-curved section) which is parallel to the longitudinal axis y, then the necking point is the midpoint of this straight section. For example, if the first channel comprises a straight section parallel to the longitudinal axis y which is 3 cm long and closer to the longitudinal axis y than any other part of the first channel, than the necking point is the midpoint of the 3 cm along the longitudinal dimension of the straight section. Similarly, if the necking point of the second channel 226 is located at a straight section (i.e. a non-curved section) which is parallel to the longitudinal axis y, then the necking point is the midpoint of this straight section. For example, if the second channel comprises a straight section parallel to the longitudinal axis y which is 3 cm long and closer to the longitudinal axis y than any other part of the second channel, than the necking point is the midpoint of the 3 cm along the longitudinal dimension of the straight section.

Alternatively, the necking point of the first channel may be closer to the longitudinal axis y than any other part of the first channel first. The necking point of the second channel may be closer to the longitudinal axis y than any other part of the second channel.

The first channel 126 may follow a first curved path 221 with only one curve between the necking point 220 and the rear end 216 of the first channel 126. The first channel 126 may follow a second curved path 222 with only one curve between the necking point 220 and the front end 215 of the first channel 126. The first curved path 221 of the first channel 126 may have a steeper curvature compared to the second curved path 222 of the first channel 126. Examples of such channel configuration is shown in Fig. 7, 8 and 9.

Likewise, the second channel 226 may follow a first curved path 221 with only one curve between the necking point 220 and the rear end 216 of the second channel 226. The second channel 226 may follow a second curved path 222 with only one curve between the necking point 220 and the front end 215 of the second channel 226. The first curved path 221 of the second channel 226 may have a steeper curvature compared to the second curved path 222 of the second channel 226. Examples of such channel configuration is shown in Fig. 7, 8 and 9.

The first and second channel 126, 226 may have a perimeter which delimits each channel against the absorbent layer surrounding it. Each channel may have a first and a second longitudinal edge on each side along its length. The first longitudinal edge 241 of the first channel 126 is formed towards the first longitudinal side edge 118 of the absorbent layer 117 and the second longitudinal edge 242 of the first channel 126 is formed towards the longitudinal axis y. The first longitudinal edge 243 of the second channel 226 is formed towards the second longitudinal side edge 218 of the absorbent layer 117 and the second longitudinal edge 244 of the second channel 226 is formed towards the longitudinal axis y. The middle between the first and second longitudinal edges of the first channel is halfway between the first and second longitudinal edge as measured at a given location of the first channel by taking the shortest distance between the first and second longitudinal edges at this location (e.g. at the necking point of the first channel). Likewise, the middle between the first and second longitudinal edges of the second channel is halfway between the first and second longitudinal edge as measured at a given location of the second channel by taking the shortest distance between the first and second longitudinal edges at this location (e.g. at the necking point of the second channel).

The first and second longitudinal edge 241, 242, the front end and the rear end 215, 216 of the first channel 126 together form the perimeter of the first channel 126. The first and second longitudinal edge 243, 244, the front end and the rear end 215, 216 of the second channel 226 together form the perimeter of the second channel 226.

The first and second channel 126, 226 are spaced apart from each other at their necking point 220 by a first distance L1. The distance L1 is measured between the middle of the first channel 126 at the first channel's necking point 220 and the middle of the second channel 226 at the second channel's necking point 220.

The absorbent layer 117 has a first transverse width W1 extending from the first longitudinally extending side edge 118 to the second longitudinally extending side edge 218 of the absorbent layer 117 across the necking point of the first and second channel.

The distance L1 is from 30 mm to 50 mm, or from 32 mm to 45 mm, or from from 35 mm to 45 mm.

The first transverse width W1 may be from 50 mm to 120 mm, or from 60 mm to 115 mm, or from 70 mm to 115 mm, or from 80 mm to 115 mm.

The first and second channel 126, 226 are spaced apart from each other at their rear ends 216 by a second distance L2. The second distance L2 is measured between the middle of the first channel 126 at the first channel's rear end and the middle of the second channel 226 at the second channel's rear end. The second distance L2 may be from 48 mm to 70 mm, or from 50 mm to 65 mm, or from 50 mm to 60 mm. The second distance L2 is larger than first distance L1.

The absorbent layer 117 has a second transverse width W2 extending from the first longitudinally extending side edge 118 to the second longitudinally extending side edge 218 of the absorbent layer 117 across the rear ends 216 of the first and second channel 126, 226. The second transverse width W2 may be from 50 mm to 120 mm, or from 60 mm to 115 mm, or from 70 to 115 mm, or from 80 to 115 mm.

The second transverse with W2 may the equal to the first transverse width W1. Alternatively, the second transverse width W2 may be larger than the first transverse width W1. The second transverse width W2 may be at least 10% larger, or at least 15% larger, or at least 20% larger, or at least 25% larger than the first transverse width W1. The second transverse width W2 may not be more than 60%, or not more than 50%, or not more than 40%, or not more than 30% larger than the first transverse width W1.

The ratio of the second transverse width W2 to the second distance L2 is from 1.5 to 2.8.

The ratio of the first transverse width W1 to the first distance L1 is higher than the ratio of the second transverse width W2 to the second distance L2.

The ratio of L2 to L1 is from 1.2 to 2.5, or from 1.4 to 2.2, or from 1.5 to 2.0.

The first and second channel may both extend across the transverse axis x of the absorbent pant. They may be provided such that the center of each of the first and second channel is provided offset from the lateral axis x towards the front waist edge. For example, the center of each of the first and second channel may be provided from 30% to 48%, or from 35% to 44% from the front waist edge of the pant based on the overall length L4 of the pant.

The first and second channel may substantially be mirror images of each other and may have substantially no offset to each other along the longitudinal axis. "Substantially" mirror images and "substantially" no offset means to include insignificant deviations which may, for example be due to variations of a high-speed manufacturing process variations.

"Substantially" no offset includes an offset of up to 10%, or up to 5% based on the length of the channel as measured along the longitudinal axis along a straight line from the front end to the rear end of the channel. The first and second channel may be mirror images of each other and may have no offset to each other along the longitudinal axis.

The first and second channel may have substantially the same length as measured along the longitudinal axis along a straight line from the front end to the rear end of the channel. "Substantially" the same length includes deviations in length of up to 10%, or up to 5% based on the length of the longer channel. The first and second channel may have the same length.

The absorbent layer may have no other channels than the first and second channel.

With the first distance L1 being from 30 mm to 50 mm, the ratio of the second transverse width W2 to the second distance L2 being from 1.5 to 2.8, the ratio of the second length L2 to first length L1 being from 1.2 to 2.5 and the ratio of W1 to L1 being higher than the ratio of W2 to L2, these dimensions and ratios in combination define an absorbent layer wherein the channels are configured such that the central chassis of absorbent pant has an improved fit. As already explained above, when the pant is being pulled up between the legs of the wearer upon application onto a wearer, the central chassis tends to fold in order to accommodate between the relatively narrow space between the legs. Having the absorbent layer and the first and second channel being configured in the claimed manner, helps to prevent that the central chassis folds too narrowly, both in the crotch and towards the rear waist region.

The first and second channel are spaced apart from each other across their necking point relatively widely compared to known channel configurations. If the channels are arranged in closer proximity to each other, the central chassis will tend to fold into a relatively narrow U-shape between the legs of the wearer, which may lead to a wider gap between the absorbent core and the body of the wearer in the crotch. This increases the risk of leakage due to reduced contact of the central chassis with the body of the wearer and due to the narrow fold which leads to bunching of the absorbent core between the wearer's legs.

As the necking point is not provided at the center of the channel but, preferably, between the center and the rear end of the channel, the fit is further improved. With the necking point being offset from the center towards the rear end, the first curved path between the necking point and the rear end of each of the first and second channel can follow a stepper curvature compared to the second curved path between the necking point and the front end of the first and second channel.

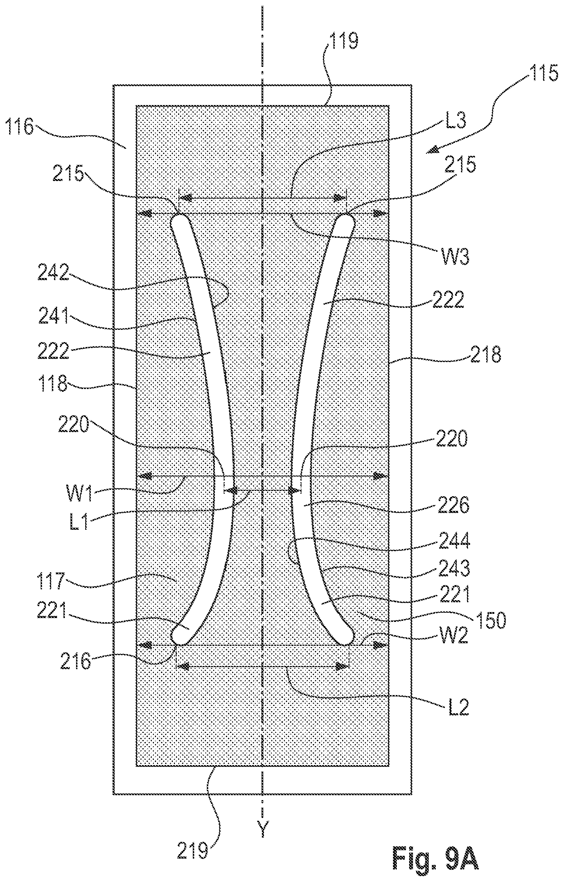

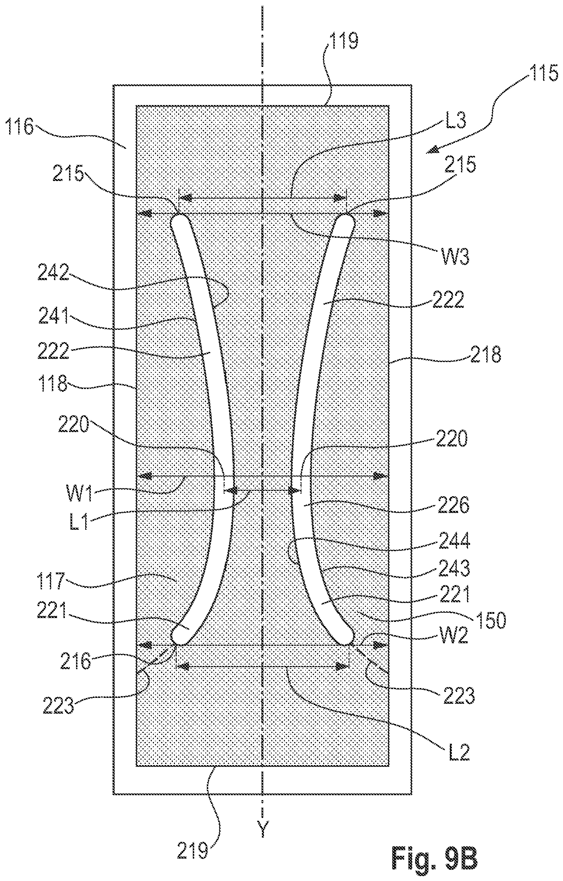

As said, the absorbent layer -and thus the central chassis as a whole- tends to fold inwardly along the channels. For pants, pulled awards on the wearer into their final wearing position, this folding typically extends beyond the ends of the channel into the absorbent layer towards the front and rear edges of the absorbent layer. Having a steeper curvature along the first curved path towards the rear end of the channel results in the fold lines continuing into the absorbent layer beyond the channel rear end being directed towards the longitudinally extending side edges of the absorbent layer, see dotted line 223 in Fig. 9, which illustrates the path of a fold line extending beyond the end of the first and second channel. This improves surface coverage of the central chassis with the body of the wearer in the rear area towards the rear belt. Especially if the first and second channel do not overlap with the rear belt, such configuration is beneficial, given that at least a portion of the dotted line shown in Fig. 9 will not be overlaid by the rear belt.

Moreover, the absorbent layer 117 is often not homogeneous outside the first and second channel. I.e. some areas of the absorbent layer have more absorbent material relative to other areas, leading to a profiled distribution of absorbent material.

The absorbent layer may comprise less absorbent material per surface area in the region towards the rear edge of the absorbent layer. For example, the area of the absorbent layer which is formed in 20% of the longitudinal dimension of the absorbent layer adjacent the rear edge of the absorbent layer, based on the total length of the absorbent layer as measured from the front edge to the rear edge along the longitudinal axis, may have less than 10%, or less than 8%, by weight of the total amount of absorbent material of the absorbent layer.

In such configurations, where the rear portion of the absorbent layer has relatively little amount of absorbent material, the absorbent core -and thus the central chassis as a whole- is more prone to bunching and folding. Therefore, it is especially beneficial to influence the formation of fold lines to some extent. As explained above, configuring the first and second channel in the claimed manner helps to facilitate the formation of folds beyond the rear end of the first and second channel such that the folds tend to form towards the longitudinally extending side edges of the absorbent layer.

Moreover, having the ratio of the first distance L1 to the second distance L2 being from 1.2 to 2.5, the ratio of the first transverse width W1 of the absorbent layer across the necking point of the first and second channel to the first distance L1 being higher than the ratio of the second transverse width W2 of the absorbent layer across the rear ends of the first and second channel to the second distance L2, results in a curvature of the first and second channel which provides improved body surface coverage and improved fit of the central chassis towards rear belt. As the second distance L2 between the first and second channel at the channels' rear ends is substantially wider than the first distance L1 at the channels' necking point, the absorbent core, and thus of the central chassis, stays wider and, overall, can reduce the inboard folding of the central chassis. This leads to wider, better body surface coverage at the buttocks of the wearer, and increases of comfort and fit.

The first and second channel 126, 226 are spaced apart from each other at their front ends 215 by a third distance L3. The third distance L3 is measured between the middle of the first channel 126 at the first channel's front end and the middle of the second channel 226 at the second channel's front end. The third distance L3 may be from 48 mm to 70 mm, or from 50 mm to 65 mm, or from 50 mm to 60 mm. The third distance L3 may be larger than the first distance L1. The third distance L3 may be equal to the second distance L2. Alternatively, L2 and L3 may not differ from each other by more than 30%, or not more than 20%, or not more than 10%, or not more than 5% based on the longer distance.

The absorbent layer 117 has a third transverse width W3 measured from the first longitudinally extending side edge to the second longitudinally extending side edge of the absorbent layer across the front ends 215 of the first and second channel 126, 226. The third transverse width W3 may be from 85 mm to 120 mm, or from 90 mm to 115 mm. The third transverse with W3 may be equal to or may be wider than the first transverse width W1. The third transverse width W3 may be equal to, smaller than, or larger than the second transverse width W2.

The ratio of the third transverse width W3 to the third distance L3 may be from 1.5 to 2.8.

The ratio of the first transverse width W1 to the first distance L1 may be higher than the ratio of the third transverse width W3 to the third distance L3.

The ratio of L3 to L1 may be from 1.2 to 2.5, or from 1.4 to 2.2, or from 1.5 to 2.0.

If the ratio of the third transverse width W3 to the third distance L3 is from 1.5 to 2.8, the ratio of the third length L3 to first length L1 is from 1.2 to 2.5 and the ratio of W1 to L1 is higher than the ratio of W3 to L3, this provides an absorbent layer wherein the channels are configured such that the central chassis of disposable absorbent pant has a further improved fit.

Moreover, if the ratio between the first distance L1 and the third distance L3 is from 1.2 to 2.5, and, further, if the ratio of the first transverse width W 1 of the absorbent layer across necking point of the first and second channel to the first distance L1 is higher than the ratio of the third transverse width W3 of the absorbent layer across the front ends of the first and second channel to the third distance L3, this provides a curvature of the first and second channel which can improve body surface coverage and fit of the central chassis towards the front belt. If the third distance L3 between the first and second channel at the channels' rear ends is substantially wider than the first distance L1 at the channels' necking point, the absorbent core, and thus of the central chassis, stays wider and, overall, can reduce the inboard folding of the central chassis. This leads to wider body surface coverage towards the front waist region of the wearer, thus increase comfort and fit.

The first and second channel each may have a width w_{C} of at least 3 mm, or from 3 mm to 15 mm, or from 5 mm to 12 mm, or from 5 mm to 10 mm as measured along the shortest path from the first to the second longitudinal edge of the first and second channel, respectively. The width may be the same along the length of each of the first and second channel. Alternatively, the width may vary across the length of the first and second channel, e.g. the width may vary from 3 mm to 15 mm, or from 5 mm to 12 mm, with the average width being from 5 to 10 mm. The width of the first and second channel, like all other dimensions and ratios provided herein, are to be measured on a dry product.

The average width of each of the first and second channel may be from 3% to 15%, or from 4% to 12%, or from 5% to 10% of the width of the absorbent layer (if the absorbent layer does not have constant width, i.e. if it does not have a rectangular shape, the width to be taken into account is the average width of the absorbent layer as measured in those areas of the absorbent layer which are provided with the first and second channel. I.e. areas towards the front and rear edges 119 and 219 of the absorbent layer, where neither the first nor the second channel are provided, are not taken into account for calculating the average width of the absorbent layer.

The first and second channel may each be permanent. By permanent, it is meant that the integrity of the channels is substantially maintained both in dry state and wet state, i.e. the channels are substantially resistant to the effects of wetting (e.g., structure is maintained by materials that are insoluble in water), and substantially withstand mechanical stresses in the materials caused by swelling of superabsorbent polymer particles, pressure within the structure resulting therefrom, and the wearer's body movements. However, the permanent first and second channel may reduce in width wc upon wetting due to swelling of the absorbent material (such as superabsorbent polymer particles) which are provided along the perimeter of the first and second channel.

Permanent channels may be formed by immobilizing the superabsorbent polymer particles on a substrate layer, such as by applying a hot-melt adhesive material over the absorbent layer.

The absorbent layer may be provided between a first substrate layer 116 positioned towards the backsheet of the central chassis, and a second substrate layer 116' positioned towards the topsheet of the central chassis. The first and second substrate layer may each comprise or consist of a nonwoven web.

The first and second channel may each be formed as permanent channels by permanently bonding of the first substrate layer and the second substrate layer together along the first and second channels, respectively, thereby forming areas that separate and contain superabsorbent polymer particle deposits (and other absorbent material, if present in the absorbent layer) and thereby define the channels therethrough. Adhesive may be used to bond (e.g. by direct bonding with no additional materials provided between the first and second substrate layer in the bonded area except for the adhesive) the first and second substrate layers 116, 116' together along the channels, but it is possible to directly bond the substrate layers together (e.g. by direct bonding with no additional materials provided between the first and second substrate layer in the bonded area except for a possible adhesive) via other means, for example, ultrasonic bonding, pressure bonding, thermal bonding or combinations thereof (including combinations with adhesive bonding). The first and second substrate layer may be continuously bonded or intermittently bonded along the channels.

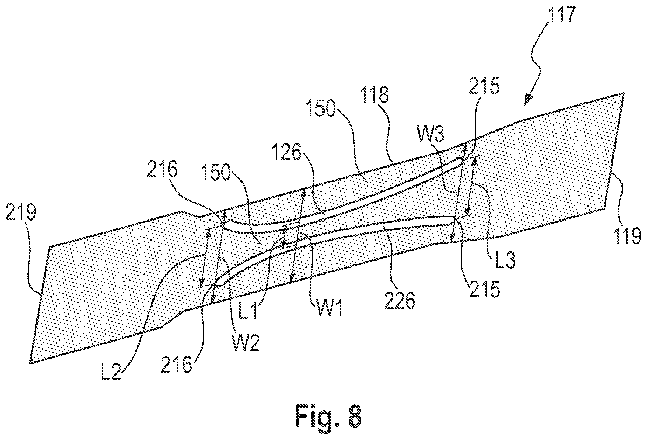

The first and second channel 126, 226 located in the absorbent layer 117 may divide the absorbent layer into three sections at least in the crotch region 123. As exemplified in Figs. 2A, 2B and 8, the channels may be present in the crotch region of the absorbent layer. The two channels may extend, as measured along the longitudinal axis y along a straight line from the channel front end 215 to the channel rear end 216, longitudinally along from 40% to 70%, or from 45% to 65%, or from 50% to 60% of the total length of the absorbent layer, as measured along the longitudinal axis y from the front edge 119 to the rear edge 219. The first and second channel may be present only in the crotch region 123.

When present only in the crotch region, the first and second channel may extend over the whole longitudinal dimension of the crotch region, or, if the first and second channel are shorter in longitudinal length than the crotch region, they may extend in only part of the crotch region. The first and second channel 126, 226 may be present in the crotch region, or part thereof, and part of the front waist region and/or part of the rear waist region. The first and second channel may be present in the front waist and crotch regions, i.e. the two channels extend from the crotch region (or part thereof) into the front waist region. The first and second channel may be present in the rear waist region and crotch regions, i.e. the channels extend from the crotch region (or part thereof) into the back region. The first and second channel 126, 226 may be mirror images of one another with respect to the longitudinal axis y of the absorbent layer 117.

It is desired that the each of the first and second channel 126, 226 does not extend all the way to the laterally extending front edge 119 and rear edge 219 of the absorbent layer 117. The absorbent layer may include, along each laterally extending edge and adjacent to said edge, a so-called "end deposit" of absorbent material, such as superabsorbent polymer particles, which is free of channels.

Adjacent to the laterally extending front edge 119 of the absorbent layer 117, such end deposits may have a respective length which is from 5% to 25%, or from 5% to 20%, or from 10% to 20% of the longitudinal dimension of the absorbent layer as measured along the longitudinal axis from the front edge 119 to the front end 215 of the first and second channel. In other words, the front end 215 of each of the first and second channel 126, 226 terminates from 5% to 25%, or from 5% to 20%, or from 10% to 20% longitudinally inboard (i.e. towards the transverse axis) from the front edge 119 of the absorbent layer.

Adjacent to the laterally extending rear edge 219 of the absorbent layer 117, such end deposits may have a respective length which is from 10% to 45%, or from 15% to 40%, or from 20% to 40% of the longitudinal dimension of the absorbent layer as measured along the longitudinal axis from the rear edge 219 to the rear end 216 of the first and second channel. In other words, the rear end 216 of each of the first and second channel 126, 226 terminates from 10% to 45%, or from 15% to 40%, or from 20% to 40%longitudinally inboard (i.e. towards the transverse axis) from the rear edge 216 of the absorbent layer.

The front end 215 of each of the first and second channel 126, 226 may be closer towards the front edge 119 of the absorbent layer than the rear end 216 is towards the rear edge 219 of the absorbent layer.

Furthermore, in order to reduce the risk of fluid leakage and run-off, it may be desired that the channels do not extend to the first and second longitudinal side edges 118, 218 of the absorbent layer 117. The absorbent layer may include, along each of the first and second longitudinally extending side edge 118, 218 and adjacent to said edge, a deposit of absorbent material, such as superabsorbent polymer particles, free of channels.

The first channel 126 is provided between the longitudinal axis y and the first side edge 118 of the absorbent layer 117. The second channel 226 is provided between the longitudinal axis y and the second side edge 218 of the absorbent layer.

Each of the first and second channel 126, 226 is curved such that the channel's necking point 220 is closer to the longitudinal axis y than the channel's front and rear ends 215, 216.

Longitudinally extended and curved channels can serve as hinge structures in the absorbent core which help enable the absorbent core to flex longitudinally and thereby conform to the wearer's anatomy along the transverse direction in the crotch region and towards/in the front and rear waist regions. Thus, the channels may contribute to imparting a comfortable and superior fit in addition to permitting improved liquid transportation and distribution. However, as described above, such flexing and folding along the channels can also have certain disadvantages when used in pants as the absorbent core may fold into a U-shape which can be too narrow if not configured in accordance with the present invention, leading in fact to reduced contact of the central chassis and the body of the wearer and bunching of the absorbent core in the crotch and towards the front and rear waist region.

The longitudinally-oriented first and second channel formed in the absorbent layer may help transport and distribute liquid (e.g., urine) along the lengths of the deposits of absorbent material, such as superabsorbent polymer particles, in the absorbent layer, and thereby help speed acquisition and absorption. However, the correspondingly-defined longitudinal areas containing or defining the deposits of superabsorbent polymer particles may develop elevated internal pressure as the particles absorb liquid, swell, and press against each other. This pressure may have a longitudinal, structural stiffening effect on the absorbent core. The internal pressure causes the absorbent layer to tend to straighten longitudinally, rather than easily curve around and beneath the wearer's lower torso as the absorbent core wraps between the wearer's legs.

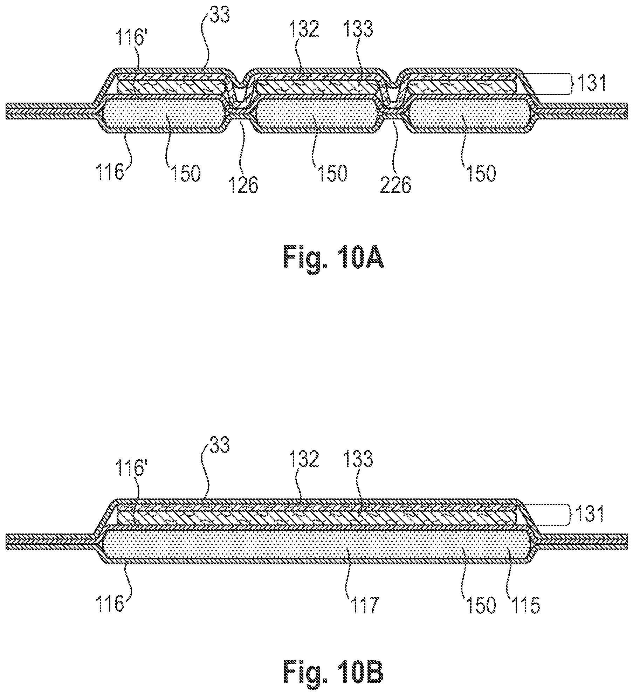

The first and second channel 126, 226 imparted in the absorbent layer 117 may be non-permanent. This enables the absorbent core to change from a first configuration when dry to a second configuration when wetted to, e.g., one-quarter, one-third, one -half, two-thirds or more of the total absorbent capacity (by weight of absorbed liquid) of the absorbent layer. For example, means or materials, such as a pressure bond or an adhesive, bonding the first substrate layer 116 to the second substrate layer 116' to form the first and second channel 126, 226 may be configured to change structure when wetted. As illustrated in Figs. 10A and 10B, an absorbent core 115 may have a first configuration when dry (e.g., Fig. 10A) and a second configuration when wetted (e.g., Fig. 10B), e.g. to more than half of its absorbent capacity. One mechanism that may be used to enable this may be a water soluble or otherwise releasable adhesive affixing the first and second substrate layer 116 and 116' together along, and thereby defining, the first and second channel 126, 226. Upon wetting and/or upon outward pressure against the first and second substrate layers 116 and 116' from the swelling deposits of superabsorbent polymer particles, the adhesive releases, and the swelling deposits of superabsorbent polymer particles are permitted to expand into the volume previously defined by (i.e. surrounding) the first and second channel 126, 226, which then may reduce in size or even disappear as suggested in Fig. 10B. This may have the effect of relieving pressure within the absorbent layer 117 and absorbent core 115, which may lessen the longitudinal stiffening effects described above. Thus, certain advantages of the first and second channel (flexibility, conformability and liquid distribution enhancement) may be enjoyed at times before the central chassis is substantially wetted, while a possible disadvantage of channels (longitudinal stiffness) may be mitigated at times after the pant has been substantially wetted.

The longitudinal dimension of each of the first and second channel 126, 226 may be notionally divided into three, four, five or more sub-lengths (i.e. subsections). Each subsection may represent at least 10%, or at least 20% of the total length of each of the first and second channel as measured along the longitudinal axis. The absorbent layer having the first and second channel provided therein, may be configured to permanently define the first and second channel along one or more of the sub-lengths, but to changeably define the channels along other of the sub-lengths, such that they reduce in size or disappear upon wetting.

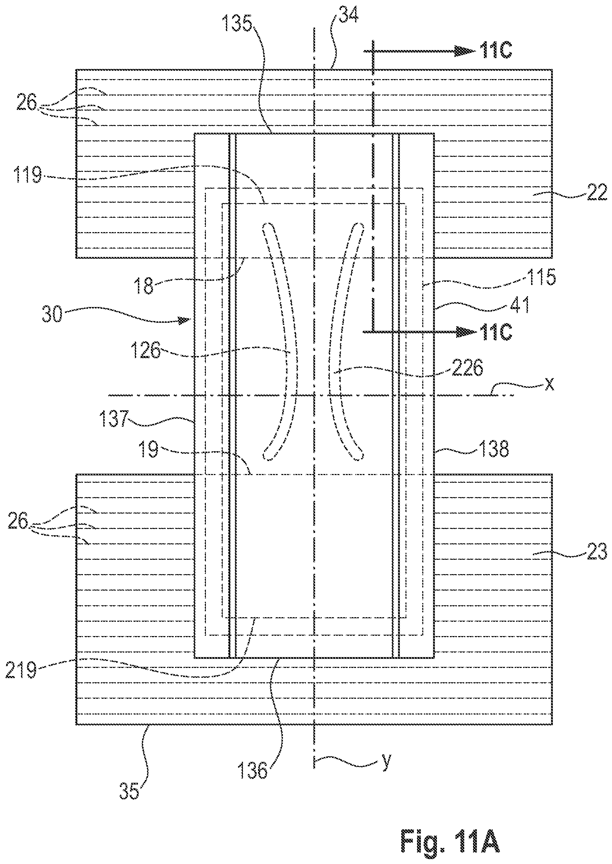

The absorbent layer 117 may extend longitudinally such that the front end 215 of the first and second channel is disposed in an overlapping configuration with the front belt 22. In that way, the front end 215 of each of the first and second channel is disposed beneath the front belt (i.e. closer to the body of the wearer) when the pant is worn. Non-limiting examples are suggested in Figs. 11A-11C. Alternatively or in addition, the absorbent layer 117 may extend longitudinally such that the rear end 216 of the first and second channel are disposed in an overlapping configuration with the rear belt 23. In that way, the rear end 216 of each of the first and second channel is disposed beneath the rear belt (i.e. closer to the body of the wearer) when the pant is worn. However, it may be preferred that the first and second channel do not overlap with the rear belt 23.

The absorbent layer, absorbent structure and/or configuration of channels may also have any features described in U.S. Pat. App. Pub. Nos. US2014/0163511; US2014/0163503; US2014/0163501; US2014/0163500; US2012/0316526; US2012/0316528; US2014/0163501; and US2014/0371701.

### Absorbent Layer

The absorbent layer may include superabsorbent polymer particles 150 alone or in combination with other materials, such as cellulose fibers. The superabsorbent polymer particles may be immobilized on the first and/or second substrate layer 116, 116' by, for example, a hot-melt adhesive material. Absorbent polymer particles suitable for use in the absorbent layer may include any superabsorbent polymer particles known from superabsorbent literature, for example such as described in Modern Superabsorbent Polymer Technology, F.L. Buchholz, A.T. Graham, Wiley 1998.

The superabsorbent polymer particles may be spherical, spherical-like, ellipsoid, or irregularly shaped, such as ovoid- shaped particles of the kind that may be obtained from inverse phase suspension polymerizations. The particles may, optionally, be agglomerated at least to some extent to form larger irregular agglomerations of particles.

The superabsorbent polymer particles may be selected from among polyacrylates and polyacrylate based materials that are internally and/or surface cross-linked, such as for example partially neutralized cross-linked polyacrylates or acid polyacrylate. Examples of absorbent polymer particles suitable in the present disclosure are described for instance in the PCT Pat. App. Nos. WO 07/047598, WO 07/046052, WO2009/155265 and WO2009/155264.

The absorbent layer may be substantially cellulose-free (also referred to as "airfelt"). "Substantially" cellulose-free, as used herein, means that the absorbent layer comprises less than 10% by weight, or less than 5% by weight, or less than 2% by weight, or less than 1% by weight of cellulose, or no cellulose (i.e. 0% by weight) based on the total weight of absorbent material in the absorbent layer. Cellulose fibers have been used as absorbent material in absorbent cores of disposable diapers. Such fibers possess absorbent properties and imparts some absorption capacity to an absorbent layer, but also may be included to provide a structural matrix to hold dispersed particles of superabsorbent polymer particles.

While inclusion of such superabsorbent polymer particles enhances absorption capacity, keeping such particles suitably dispersed may be important to prevent the particles from "gel-blocking" in use as they swell with absorbed liquid and block the passageways therebetween which allow liquid to move through deposits thereof, compromising absorption capacity. The inclusion of cellulose fibers ("airfelt") as a matrix for superabsorbent polymer particles can serve to reduce or prevent gel-blocking. However, it also imparts bulk to an absorbent layer, even before absorption of any liquids. To reduce the overall size and/or thickness of the absorbent layer, and thereby improve wearer comfort and reduce the bulkiness of the pant for purposes of packaging and shipping volume efficiency, it may be desired to construct an absorbent core using the lowest volumes of core materials possible within performance constraints. Toward this end, examples of suitable materials and constructions for a suitable absorbent structure are described in, but are not limited toWO2008/155699. Generally, these applications describe absorbent layer constructions that minimize or eliminate the need for and inclusion of airfelt in combination with particles of superabsorbent polymer particles ("substantially cellulose-free" structures). Suitable methods for forming deposits of absorbent superabsorbent polymer particles are additionally disclosed in, for example, EP 1621167 A2, EP 1913914 A2 and EP 2238953 A2.

If the absorbent core 115 comprises a first and second substrate layer 116, 116' with the absorbent layer 117 provided in between, the absorbent material, such as the superabsorbent polymer particles, may be immobilized on one or both of the first and second substrate layer.

Immobilization may be achieved by applying a hot-melt adhesive material, which holds and immobilizes the absorbent material, such as the superabsorbent polymer particles (and cellulose fibers when present), on the first and/or second substrate layer. Some hot-melt adhesive material may also penetrate into the layer of absorbent material, such as the layer of superabsorbent polymer particles, and into the first and/or second substrate layer to provide further immobilization and affixation. The hot-melt adhesive material may not only help in immobilizing the absorbent material, such as the superabsorbent polymer particles, on the substrate layer but also may help in maintaining the integrity of the first and second channel.

The hot-melt adhesive material avoids that a significant amount of absorbent material, such as superabsorbent polymer particles, migrates into the channels.

Hot-melt adhesive materials suitable for use in the present disclosure may include at least a thermoplastic polymer in combination with a plasticizer and other thermoplastic diluents such as tackifying resins and additives such as antioxidants.

Example suitable hot melt adhesive materials are described in EP 1447067 A2.

### Acquisition / distribution system

The central chassis 30 may comprise an acquisition/distribution system (ADS) 131, which is disposed between the absorbent core 115 and the topsheet 33. The acquisition/distribution system may serve as a temporary reservoir for liquid until the absorbent core can absorb the liquid, and for subsequent distribution of the liquid into the absorbent core in an efficient manner. The acquisition/distribution system may consist of a single layer or comprise multiple layers, such as an upper layer 132 provided adjacent to the topsheet 33 and facing towards the wearer's skin, and a lower layer 133 provided adjacent to the absorbent core 115 and facing the garment of the wearer. The acquisition/distribution system may be in direct contact with the absorbent core.

The ADS 131 may be free of superabsorbent polymer. The prior art discloses many types of acquisition-distribution systems, see for example WO2000/59430, WO95/10996, US5700254, WO02/067809.

The function of a lower layer 133 is typically to spread the insulting fluid liquid over a larger surface within the central chassis so that the absorbent capacity of the absorbent core can be more efficiently used. The lower layer 133 may be made of a nonwoven material based on synthetic or cellulosic fibers and having a relatively low density. The lower layer may typically have an average basis weight of from 30 to 400 g/m², in particular from 80 to 300 g/m². The lower layer may not be formed of a coherent, self-sustaining web or sheet but may be a layer with little integrity on its own.

The lower layer 133 may for example comprise at least 50%, or 60%, or 70%, or 80%, or 90% by weight of cross-linked cellulose fibers. The cross-linked cellulosic fibers may be crimped, twisted, or curled, or a combination thereof including crimped, twisted, and curled. The cross-linked cellulosic fibers provide higher resilience and therefore higher resistance to the first absorbent layer against the compression in the product packaging or in use conditions, e.g. under baby weight. This provides the central chassis with a relatively high void volume, permeability and liquid absorption, and hence reduced leakage and improved dryness.

The lower layer 133 comprising cross-linked cellulose fibers, may comprise other fibers, but this layer may advantageously comprise at least 50%, or 60%, or 70%, or 80%, or 90% or even up to 100%, by weight of the layer, of cross-linked cellulose fibers. Examples of such mixed layer of cross-linked cellulose fibers may comprise 70% by weight of chemically cross-linked cellulose fibers, 10 % by weight polyester (PET) fibers, and 20 % by weight untreated pulp fibers. In another example, the layer of cross-linked cellulose fibers may comprise 70 % by weight chemically cross-linked cellulose fibers, 20 % by weight lyocell fibers, and 10% by weight PET fibers. In another example, the layer may comprise 68 % by weight chemically cross-linked cellulose fibers, 16 % by weight untreated pulp fibers, and 16 % by weight PET fibers.

The central chassis 30 may further comprise an upper layer 132, whose function is typically to quickly acquire the fluid away from the topsheet so as to provide a good dryness for the wearer. The upper layer 132 is typically placed directly under the topsheet and directly above the lower layer 133. The upper layer 132 may typically be or comprise a non-woven material, for example a SMS or SMMS material, comprising two outer spunbonded (S) layers with one or more melt-blown (M) layers in between, or alternatively a carded chemical-bonded nonwoven. The non-woven material may, in particular, be latex bonded. Exemplary upper layers 132 are disclosed in US7786341. Carded, resin-bonded nonwovens may be used, in particular where the fibers used are solid round or round and hollow PET staple fibers (such as a 50/50 or 40/60 mix of 6 denier and 9 denier fibers).

A carded resin-bonded upper layer 132 may be stabilized by a latex binder, for example a styrene-butadiene latex binder (SB latex). Processes for obtaining such lattices are known, for example, from EP 1.49 880 (Kwok) and US 2003/0105190 (Diehl et al.). The binder may be present in the upper layer 132 in excess of 15%, or of 20% by weight, but may be present by not more than 40%, or not more than 35% by weight of the upper layer 132. SB latex is, for example, available under the trade name GENFLO^{™} 3160 (OMNOVA Solutions Inc.; Akron, Ohio).

A further layer may be used in the ADS 131 in addition to upper and lower layer 132 and 133 described above. For example, a wet-laid cellulose layer (so-called tissue) may be placed between the upper and lower layer 132 and 133 or between the lower layer 133 and the absorbent core 115. The tissue and the upper layer 132 may be of the same size or may be of different size, for example the tissue may extend further in the back of the central chassis 133 than the upper layer. An example of hydrophilic tissue is a 13 - 15 g/m² high wet strength tissue from supplier Havix. However, the ADS may only consist of the upper and the lower layer 132 and 133.

One or more of the layers of the ADS may have elongated longitudinally extending first and second openings 141 and 142, each opening having a front opening end and a rear opening end. The first and second opening may be congruent with the first and second channel 126, 226 in the absorbent layer (i.e. the first opening is congruent with the first channel and the second opening is congruent with the second channel), while being shorter than the first and second channel in longitudinal dimension. Consequently, the front opening end of the first and second opening 141 and 142 may be longitudinally offset from the front end 215 of the first and second channel 126 and 226, respectively. Thus, the front opening end of the first and second opening may be closer to the transverse axis x than the front end of the first and second channel. Alternatively, or in addition to the offset of the front end of the first and second openings relative to the front end of the first and second channel, the rear opening end of the first and second opening 141 and 142 may be longitudinally offset from the rear end 216 of the first and second channel 126 and 226, respectively. Thus, the rear opening end of the first and second opening may be closer to the transverse axis x than the rear end of the first and second channel.

In one configuration, the ADS may have an upper and a lower layer 132 and 133. The first and second opening 141 and 142 described above may be provided in the upper and the lower layer. However, it may be desirable to provide the first and second opening 141 and 142 in the lower layer 133 while leaving the upper layer 132 free of openings. The upper layer 132 may be directly attached to the absorbent core 115, e.g. to the second substrate layer 116' of the absorbent core 115, through the first and second opening in the lower layer 133. This direct attachment may be facilitated by adhesive bonding, pressure bonding, heat bonding, ultrasonic bonding, or combinations thereof.

By attaching the upper layer 132 to the absorbent core through the first and second opening 141 and 142 of the lower layer 133, the lower layer is held in position, also during use of the absorbent article. The lower layer 133 may be formed of fibers which are not consolidated into a coherent web or sheet, e.g. by air laying the fibers of the lower layer 133 without subsequently bonding the each other. In such configurations, the material of the lower layer may be more prone to shifting and moving out of place, especially during use and after the central chassis has been wetted with urine or other liquid. Therefore, attaching the upper layer to the absorbent core through the first and second openings of the lower layer is especially beneficial.

Also, by having the first and second channel being largely coextensive with the first and second opening (as said, the first and second opening may be shorter than the first and second channel) further improves transport and distribute liquid (e.g., urine) to the absorbent core and along the lengths of the deposits of absorbent material, such as superabsorbent polymer particles, in the absorbent layer, and thereby help speed acquisition and absorption.

### First and second elasticized belt

The disposable absorbent pant of the present invention comprises an elasticized front belt 22 provided in the front waist region and an elasticized rear belt 23 provided in the rear waist region. The front and rear belt 22 and 23 each have a body-facing surface and a garment-facing surface. The body-facing surface will face towards the skin of the wearer in use of the disposable pant and the garment-facing surface will face away from the skin of the wearer during use of the disposable pant and towards the garment of the wearer.

The front belt 22 has a transversally extending front waist edge 34 and the rear belt 23 has a transversally extending rear waist edge 35. The front and rear belt 22 and 23 each have a first and second longitudinally extending side edge 230 and 231. The first side edge 230 of the front belt 22 is joined to the first side edge 230 of the rear belt 23 and the second side edge (231) of the front belt (22) is joined to the second side edge 231 of the rear belt 23 at side seams 24 to form a waist opening and two leg openings 15. The waist opening is formed in conjunction by the front and rear waist edge.

Each of the front and rear belt 22 and 23 may be formed of an inner layer 25a, an outer layer 25b and a plurality of elastic members 26, such as elastic strands, disposed between the inner layer and the outer layer. The plurality of elastic members, may be provided such that they extend in transverse direction and the individual elastic members are longitudinally spaced apart from each other.

The pant may have the front and rear belt 22 and 23 being longitudinally separated from each other such that there is a gap between the front and rear belt along the longitudinal axis y and the central chassis 30 is provided in between the front and rear belt 22 and 23 along the longitudinal axis y. In such configurations, the central chassis 30 overlaps the front and rear belt 22 and 23 and is attached to the front belt 22 in the overlapping regions, i.e. along and/or adjacent the central chassis' laterally extending chassis front edge 135 and attached to the rear belt 23 along and/or adjacent its laterally extending chassis rear edge 136. Attachment may be facilitated by adhesive bonding, pressure bonding, heat bonding, ultrasonic bonding, or combinations thereof. Further, in such configurations, the central chassis is attached to the body-facing surface of the front and rear belt 22 and 23. In such configurations, the elasticized front belt 22 has a first transversally extending first lower edge 18 disposed between the front waist edge 34 and the lateral axis x, and the elasticized rear belt 23 has a transversally extending second lower edge 19, disposed between the rear waist edge 35 and the lateral axis x. If the front and rear belt 22 and 23 comprise an inner and an outer layer, the first and second lower edges 21 may be formed by one or both of the inner and outer layers.

The pant has an overall length L4 as measured from the front waist edge 34 to the rear waist edge 35 along the longitudinal axis y. The overall length L4 is measured prior to attaching the front and rear belt to each other along side seams 24, or after the side seams have been torn open, and the pant has been stretched out flat on an even surface, such as a table.

For pants having the front and rear belt not extending through the crotch region but having a first transversally extending first lower edge 18 at the front belt and a second lower edge 19 at the rear belt, the crotch region is defined in between the first and second lower edge 18 and 19 along the longitudinal axis y. I.e. the crotch region is formed by those portions of the central chassis which do not overlap with the front or rear belt. In such pants, the pant has a crotch region length L5 as measured from the first lower edge 18 of the front belt to the second lower edge 19 of the rear belt.

If the first and/or second lower edge 18 and 19 are not parallel to the transverse axis x, the crotch region length L5 is measured at the longitudinal axis.

In order to improve proper fit of the pant, especially in the crotch region, the ratio of the overall length L4 to the crotch region length L5 may be from 1.9 to 2.6, or from 2.0 to 2.5. Such ratios ensure that the crotch region has an appropriate length versus the overall length L4 of the disposable pant, thus helping a close contact of the central chassis to the body of the wearer in the crotch region and good positioning of the front and rear belt across the waist, hips, belly and back of the wearer for improved fit.

The absorbent layer 117 of the absorbent core 115 may overlap with the front and rear belt.

The rear end 216 of each of the first and second channel 126, 226 may not overlap with the rear belt 23. Thereby, the first and second channels cannot act as hinges or fold lines in the area of the absorbent layer, which overlaps the rear belt. Consequently, the transversally extending elastic members are not contracted by the pulling forces of an absorbent layer which folds along the first and second channel. The rear end 216 may be spaced away (towards the transverse axis x) from the second lower edge 19 by from 14 mm to 25 mm, or from 15 mm to 22 mm. This range has been found advantageous irrespective of the overall length L5 of the crotch region. The crotch region length L5 may be from 170 mm to 250 mm. By this spaced configuration, the surface coverage of the skin at the buttocks of the wearer is improved. Due to the offset of the first and second channel's rear end from the second lower edge 19, the U-shaped folding which the absorbent core -and the central chassis as a whole - tends to adopt upon pulling up the disposable pants to put the pant in place on the wearer, stops at a distance below the second lower edge 19, thereby helping to properly fit the central chassis beneath the rear belt on the skin of the wearer without any undesired and uncomfortable bunching and folding of the central chassis beneath the rear belt.

The front end 215 of each of the first and second channel 126, 226 may overlap with the front belt 22. Given that the body surface of the wearer is typically smaller in the area where the crotch region migrates to the belly of the wearer, and thus at the lower portion of the front belt compared to the buttocks area, folding along the front ends 215 of each of the first and second channel does not negatively impact fit but rather can help to facilitate snug fit on the wearer, also when the front ends 215 overlap with the front belt. The front end 215 of each of the first and second channel may overlap the front belt by from 3 mm to 25 mm, or from 5 mm to 20 mm.

Alternatively to the disposable pant configuration described above, the front and rear belt may extend into the crotch region such that the pant does not have a first and second lower edge 18 and 19. In such pants, the central chassis will be covered by one or more layers of the front and rear belt 22 and 23 across the complete longitudinal dimension of the central chassis. One or more layers may continuously form the front and rear belt 22 and 23 in such configurations. For these kinds of disposable pants, the "crotch region" is the portion through which the lateral axis (herein, axis x) passes, and which extends longitudinally one-sixth of the overall length L4 of the pant frontward and rearward of the lateral axis. Accordingly, the front waist region includes the front one-third of the overall length L4 of the pant; the crotch region includes the middle one-third of the overall length L4 of the pant; and the rear waist region includes the rear one-third of the overall length L4 of the pant.

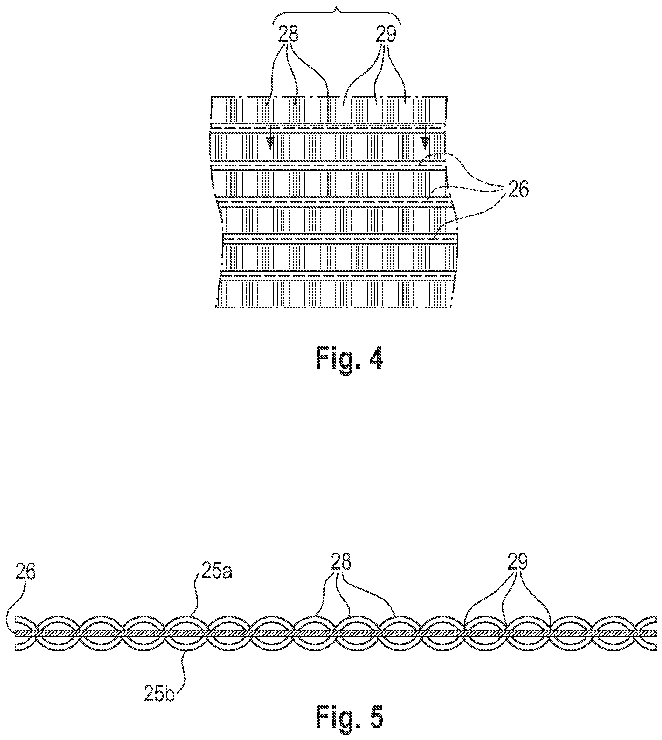

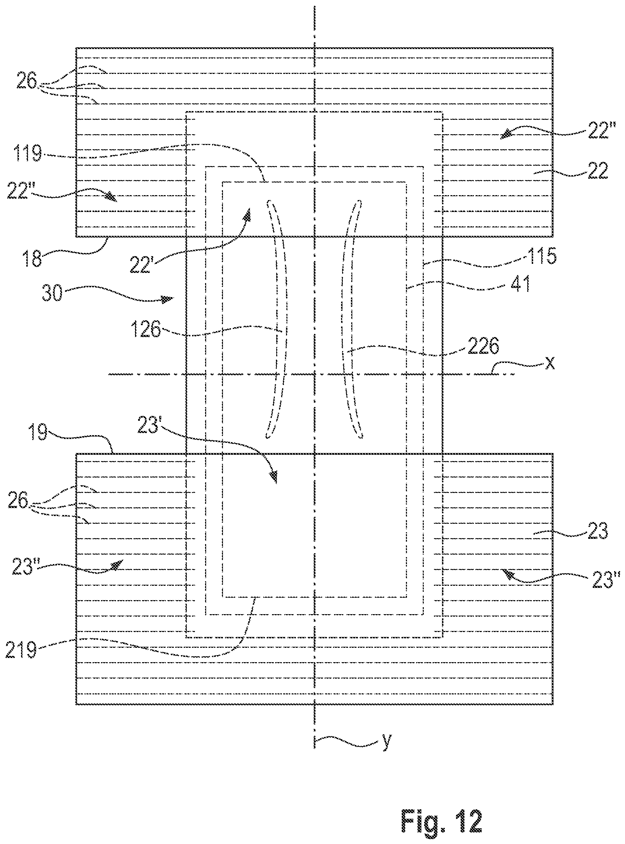

Referring e.g to Fig. 12, elastic members such as elastic strands 26 may be configured within the front and/or rear belt such that they are present in lower side zones 22", 23" (i.e. the zones lying left and right outside of a central zone 22' and 23' of the front and rear belt which is overlaid by the central chassis) of the front and rear belt, but not present in part or all of the lower central zones 22', 23' that overlie the central chassis 30. Thus, one or both belt may be configured such that one, more than one, or all of the layers that sandwich the elastic strands 26 are present in lower central zones 22', 23' of the front and rear belt 22, 23, such as nonwoven layers 25a, 25b (see Fig. 3), without elastic stretch enabled by the presence of pre-strained elastic members and ruffles of laterally gathered material. (Notably, in Fig. 3, "X" designates the lateral direction in general, not the lateral axis.) In the central zones 22', 23' that overlie the central chassis, the nonwoven layer(s) of one or both belts 22, 23 may be disposed and affixed to the central chassis material(s) (such as the backsheet) such that they overlie the central chassis in laterally extended condition, i.e. , they do not have longitudinal ruffles or rugosities (e.g. , ruffles or rugosities 27, illustrated in Figs. 4 and 5) that would otherwise be imparted by lateral contraction of pre-strained, sandwiched lateral elastic strands. In this configuration, the fully extended belt layer material(s) overlying the central chassis 30 in lower central zones 22', 23' , being without longitudinal ruffles and thereby being unable or less able to elastically stretch laterally as compared to the other elasticized, ruffled zones of the belt portions, are configured to provide greater resistance to lateral expansion, supplementing that of the central chassis materials and helping to support and restrain the ends of absorbent layer 117. This feature may be combined with any of the channel configurations described above, for potentially synergistic effects in reducing folding and bulging of the absorbent layer 117 as described above.

PCT/CN2014/094890, which describes additional examples of belt configurations having non- elasticized portions overlying the chassis.

In a further example, one or more elastic strands 26 present in the lower side zones 22", 23" may be selected (e.g. by decitex and/or tensile modulus) and/or configured (e.g. by longitudinal numerical count/unit longitudinal dimension of the belt, and/or amount of imparted pre-strain) to impart greater tensile contractive force to the front and rear belt in one or more of the lower side zones 22", 23" than in the upper zone(s) closer to the waist edges. This latter example may help enhance comfort of the pant, when worn, by providing for relatively lesser lateral contractive tensile force about the waist band areas and waist edges, and relatively greater lateral contractive tensile force with greater support, resistance to bulging of the channeled absorbent layer, and anchoring of the pant about the wearer's lower hips. Thus, one or more of the elastic strands 26 in one or both of lower side zones 22", 23" may have one or more of greater decitex, greater tensile modulus, greater number of strands 26 per unit longitudinal length of the belt, or greater amount of pre-strain, than one or more of the elastic strands 26 in the upper zone(s) closer to the waist edges in the same front or rear belt. This feature may be incorporated alone, or in combination with, the inclusion of non-elasticized central zone(s) 22', 23' of the belt described immediately above.

## Claims

1. A disposable absorbent pant, having a longitudinal axis (y), a lateral axis (x), a front waist region, a rear waist region and a crotch region between the front and rear waist regions, the pant comprising:
A central chassis (30) extends from the front waist region through the crotch region to the rear waist region and comprises a topsheet (33), a backsheet (31) and an absorbent core (115) placed in between the topsheet (33) and the backsheet (31), the central chassis (30) having a laterally extending chassis front edge (135), a laterally extending chassis rear edge (136) and first and second longitudinally extending chassis side edges (137, 138); and
an elasticized front belt (22) provided in the front waist region and an elasticized rear belt (23) provided in the rear waist region, the front and rear belt (22, 23) each having a body-facing surface and a garment-facing surface, the front belt (22) having a transversally extending front waist edge (34), the rear belt (23) having a transversally extending rear waist edge (35), the front and rear belt (22, 23) each having a first and second longitudinally extending side edge (230, 231), with the first side edge (230) of the front belt (22) being joined to the first side edge (230) of the rear belt (23) and the second side edge (231) of the front belt (22) being joined to the second side edge (231) of the rear belt (23) at side seams (24) to form a waist opening and two leg openings (15); and
the absorbent core (115) comprising an absorbent layer (117), the absorbent layer (117) having a laterally extending front edge (119), a laterally extending rear edge (219) and first and second longitudinally extending side edges (118, 218), the absorbent layer (117) comprising a first and a second longitudinally-oriented elongate channel (126, 226) formed therein; and
the first channel (126) being provided between the longitudinal axis (y) and the first side edge (118) of the absorbent layer (117), the second channel (226) being provided between the longitudinal axis (y) and the second side edge (218) of the absorbent layer (117); and
the first and second channel (126, 226) each having a channel front end (215) towards the absorbent layer's front edge (119), a channel rear end (216) towards the absorbent layer's rear edge (219) and a center (214) which is equally spaced from the channel's front and rear ends (215, 216) across the longitudinal axis (y), each of the first and second channel (126, 226) being curved
- such that the first channel (126) is closer to the longitudinal axis (y) at a necking point than the front and rear end (216) of the first channel (126), and
- such that the second channel (226) is closer to the longitudinal axis (y) at a necking point (220) than the front and rear end (216) of the second channel (226),
the first and second channel (126, 226) being spaced apart from each other at their necking point (220) by a first distance L1, wherein the distance L1 is from 30 mm to 50 mm
the absorbent layer (117) having a first transverse width W1 extending from the first longitudinally extending side edge (118) to the second longitudinally extending side edge (218) across the necking point of the first and second channel;
the first and second channel (126, 226) being spaced apart from each other at their rear ends (216) by a second distance L2, the absorbent layer (117) having a second transverse width W2 extending from the first longitudinally extending side edge (118) to the second longitudinally extending side edge (218) across the rear ends (216) of the first and second channel (126, 226),
wherein the ratio of W2 to L2 is from 1.5 to 2.8,
wherein the ratio of W1 to L1 is higher than the ratio of W2 to L2, and
wherein the ratio of L2 to L1 is from 1.2 to 2.5.

2. The disposable absorbent pant of claim 1, wherein the necking point (220) of the first channel (126) is spaced from 5% to 30% away from the center (214) of the first channel (126) towards the rear end (216), based on the total length of the first channel (126), as measured along a straight line from the front end (215) to the rear end (216) of the first channel (126); and wherein the necking point (220) of the second channel (226) is spaced from 5% to 30% away from the center (214) of the second channel (226) towards the rear end (216), based on the total length of the second channel (226), as measured along a straight line from the front end (215) to the rear end (216) of the second channel (226).

3. The disposable absorbent pant of any of the preceding claims, wherein the first channel (126) and the second channel (226) are not closer to the longitudinal axis (y) at any other location than at their necking point (220).

4. The disposable absorbent pant of any of the preceding claims, wherein the first channel (126) follows a first curved path (221) with only one curve between the necking point (220) and the rear end (216) of the first channel (126), and the first channel (126) follows a second curved path (222) with only one curve between the necking point (220) and the front end (215) of the first channel (126), wherein the first curved path (221) of the first channel (126) has a steeper curvature compared to the second curved path (222) of the first channel (126); and
wherein the second channel (226) follows a first curved path (221) with only one curve between the necking point (220) and the rear end (216) of the second channel (226), and the second channel (226) follows a second curved path (222) with only one curve between the necking point (220) and the front end (215) of the second channel (226), wherein the first curved path (221) of the second channel (226) has a steeper curvature compared to the second curved path (222) of the second channel (226).

5. The disposable absorbent pant of any of the preceding claims, wherein the absorbent layer (117) comprises less than 10% by weight of absorbent material, based on the total amount of absorbent material in the absorbent layer, in an area extending from the rear edge (219) of the absorbent layer towards the lateral axis (x) and spanning 20% of the longitudinal dimension of the absorbent layer (117), based on the total length of the absorbent layer (117) as measured from the front edge (119) to the rear edge (219) of the absorbent layer (117) along the longitudinal axis (y).

6. The pant of any of the preceding claims, wherein the first and second channel (126, 226) are spaced apart from each other at their front ends (215) by a third distance L3, the absorbent layer (117) having a third transverse width W3 extending from the first longitudinally extending side edge (118) to the second longitudinally extending side edge (218) across the front ends (215) of the first and second channel (126, 226),
wherein the ratio of W3 to L3 is from 1.5 to 2.8,
wherein the ratio of W1 to L1 is higher than the ratio of W3 to L3, and
wherein the ratio of L3 to L1 is from 1.2 to 2.5, or from 1.4 to 2.2, or from 1.5 to 2.0.

7. The pant of claim 5, wherein L2 and L3 do not differ from each other by more than 30% based on the longer distance.

8. The pant of any of the preceding claims, wherein the ratio of W1 to L1 is from 2.5 to 4.5, preferably from 2.8 to 4.0, and even more preferably from 3.0 to 3.8.

9. The pant of any of the preceding claims, wherein the elasticized front belt (22) has a first transversally extending first lower edge (18) disposed between the front waist edge (34) and the lateral axis (x), and the elasticized rear belt (23) has a transversally extending second lower edge (19), disposed between the rear waist edge (35) and the lateral axis (x), and the central chassis (30) is joined to the body-facing surface of the front belt (22) adjacent to its chassis front edge (135) and joined to the body-facing surface of the rear belt (23) adjacent to its chassis rear edge (136).

10. The pant of claim 9, wherein the pant has an overall length L4 as measured from the front waist edge (34) to the rear waist edge (35) along the longitudinal axis (y), the pant further having a crotch region length L5 as measured from the first lower edge (18) to the second lower edge (19), wherein the ratio of the overall length L4 to the crotch region length L5 is from 1.9 to 2.6; or from 2.0 to 2.5.

11. The pant of claim 9 or 10, wherein the rear end (216) of the first and second channel (126, 226) is spaced from the second lower edge (19) by from 14 mm to 25 mm.

12. The pant of any of the preceding claims, wherein the first transverse width W1 of the absorbent layer (117) extending from the first longitudinally extending side edge (118) to the second longitudinally extending side edge (218) across the necking point (220) of the first and second channel (126, 226) is from 50 mm to 120 mm, or from 60 mm to 115 mm.

13. The pant of any of the preceding claims, wherein the first and second channels (126, 226) have a width of from 5 to 12 mm, or from 5 mm to 10 mm.

14. The pant of any of the preceding claims, wherein the first and second channel (126, 226) are substantially mirror images of each other with substantially no offset to each other along the longitudinal axis (y).

15. The pant of any of the preceding claims, wherein the length of each of the first and second channel (126, 226) as measured along the longitudinal axis (y) is from 40% to 70%, or from 45% to 65%, or from 50% to 60% of the total length of the absorbent layer (117), the total length of the absorbent layer (117) being measured along a straight line along the longitudinal axis (y) from the front edge (119) to the rear edge (219) of the absorbent layer (117).

16. The pant of any of the preceding claims, wherein each belt (22, 23) has an inner layer (25a), an outer layer (25b) and a plurality of elastic members (26) disposed between the inner layer and the outer layer.

17. The pant of any of the preceding claims, wherein the absorbent core (115) comprises a first substrate layer (116), such as a nonwoven web, towards the backsheet (31) and a second substrate layer (116'), such as a nonwoven web, towards the topsheet (33), and absorbent material (150) provided between first and second substrate layer (116, 116'), the absorbent material (150) forming the absorbent layer (117).

18. The pant of any of claim 17, wherein the first and second channels (126, 226) in the absorbent core are substantially free of absorbent material (150).

19. The pant of any of claims 17 or 18, wherein the absorbent material (150) comprises at least 90% of superabsorbent polymer particles by total weight of the absorbent material (150) provided between the first and second substrate layer (116, 116').

20. The pant of any of claims 1 to 18, wherein the absorbent layer comprises superabsorbent polymer particles and cellulose fibers.

21. The pant of any of the preceding claims, wherein the absorbent layer (117) overlaps with the front and rear belt (22, 23).

22. The pant of any of the preceding claims, wherein the rear end (216) of the first and second channel (126, 226) does not overlap with the rear belt (23) and the rear end (216) of the first and second channel (126, 226) is spaced away from the second lower edge (19) by from 14 mm to 25 mm.

23. The pant of any of the preceding claims, wherein the central chassis (30) further comprises an acquisition/distribution system (131) interposed between the absorbent core (115) and the topsheet (33), the acquisition/distribution system (131) comprising one or more layers (132, 133), at least one of the layers (133) having longitudinally extending first and second openings (1.41, 142), each of the first and second opening (141, 142) having a front opening end and a rear opening end, the first and second openings (141, 142) being congruent with the first and second channels (126, 226) but being shorter than the first and second channels (126, 226) such that their front opening ends are longitudinally offset from the front end (215) of the first and second channel (126, 226), respectively, and their rear opening ends are longitudinally offset from the rear ends (216) of the first and second channel (126, 226), respectively.

24. The pant of claim 23, wherein the acquisition/distribution system (131) has two layers, an upper layer (132) provided adjacent to the topsheet (33) and a lower layer (133) provided adjacent to the absorbent core (115), wherein the longitudinally extending first and second openings (141, 142) are provided in the lower layer (133) and the upper layer (132) is free of openings, the upper layer (132) being directly attached to the absorbent core (115) through the first and second openings (141, 142) of the lower layer (133).

## Patentansprüche

1. Einwegabsorptionshöschen mit einer Längsachse (y), einer Querachse (x), einem vorderen Taillenbereich, einem hinteren Taillenbereich und einem Schrittbereich zwischen dem vorderen und dem hinteren Taillenbereich, wobei das Höschen umfasst:
eine zentrale Grundeinheit (30), die sich von dem vorderen Taillenbereich durch den Schrittbereich zu dem hinteren Taillenbereich erstreckt und eine Oberschicht (33), eine Unterschicht (31) und einen Absorptionskern (115), der zwischen der Oberschicht (33) und der Unterschicht (31) platziert ist, erstreckt, wobei die zentrale Grundeinheit (30) einen quer verlaufenden Grundeinheit-Vorderrand (135), einen quer verlaufenden Grundeinheit-Hinterrand (136) und einen ersten und einen zweiten längs verlaufenden Grundeinheit-Seitenrand (137, 138) aufweist; und
einen elastifizierten vorderen Bund (22), der im vorderen Taillenbereich bereitgestellt ist, und einen elastifizierten hinteren Bund (23), der im hinteren Taillenbereich bereitgestellt ist, wobei der vordere und der hintere Bund (22, 23) jeweils eine körperseitige Oberfläche und eine kleidungsseitige Oberfläche aufweisen, wobei der vordere Bund (22) einen quer verlaufenden vorderen Taillenrand (34) aufweist, der hintere Bund (23) einen quer verlaufenden hinteren Taillenrand (35) aufweist, der vordere und der hintere Bund (22, 23) einen ersten und einen zweiten Längsseitenrand (230, 231) aufweist, wobei der erste Seitenrand (230) des vorderen Bundes (22) mit dem ersten Seitenrand (230) des hinteren Bundes (23) verbunden ist und der zweite Seitenrand (231) des vorderen Bundes (22) mit dem zweiten Seitenrand (231) des hinteren Bundes (23) an Seitennähten (24) verbunden ist, um eine Taillenöffnung und zwei Beinöffnungen (15) zu bilden; und
der Absorptionskern (115) eine Absorptionsschicht (117) umfasst, wobei die Absorptionsschicht (117) einen quer verlaufenden vorderen Rand (119), einen quer verlaufenden hinteren Rand (219) und einen ersten und einen zweiten Längsseitenrand (118, 218) aufweist, wobei die Absorptionsschicht (117) einen ersten und einen zweiten längs ausgerichteten verlängerten Kanal (126, 226) umfasst, der darin ausgebildet ist; und
wobei der erste Kanal (126) zwischen der Längsachse (y) und dem ersten Seitenrand (118) der Absorptionsschicht (117) bereitgestellt ist, wobei der zweite Kanal (226) zwischen der Längsachse (y) und dem zweiten Seitenrand (218) der Absorptionsschicht (117) bereitgestellt ist; und
wobei der erste und der zweite Kanal (126, 226) jeweils ein vorderes Kanalende (215) in Richtung des vorderen Randes (119) der Absorptionsschicht, ein hinteres Ende (216) in Richtung des hinteren Randes der Absorptionsschicht (219) und eine Mitte (214) aufweisen, die gleich weit von dem vorderen und hinteren Ende des Kanals (215, 216) über die Längsachse (y) beabstandet ist, wobei jeder des ersten und des zweiten Kanals (126,226) gekrümmt ist
- sodass der erste Kanal (126) an einem Halspunkt näher an der Längsachse (y) liegt als am vorderen und hinteren Ende (216) des ersten Kanals (126) und
- sodass der zweite Kanal (226) an einem Halspunkt (220) näher an der Längsachse (y) liegt als am vorderen und hinteren Ende (216) des zweiten Kanals (226),
wobei der erste und der zweite Kanal (126, 226) an ihrem Halspunkt (220) um einen ersten Abstand L1 voneinander beabstandet sind, wobei der Abstand L1 von 30 mm bis 50 mm beträgt,
die Absorptionsschicht (117) eine erste Querbreite W1 aufweist, die sich vom ersten Längsseitenrand (118) zum zweiten Längsseitenrand (218) über den Halspunkt des ersten und des zweiten Kanals erstreckt;
wobei der erste und der zweite Kanal (126, 226) an ihren hinteren Enden (216) um einen zweiten Abstand L2 voneinander beabstandet sind, wobei die Absorptionsschicht (117) eine zweite Querbreite W2 aufweist, die sich vom ersten Längsseitenrand (118) zum zweiten Längsseitenrand (218) über die hinteren Enden (216) des ersten und des zweiten Kanals (126, 226) erstreckt,
wobei das Verhältnis von W2 zu L2 von 1,5 bis 2,8 beträgt;
wobei das Verhältnis von W1 zu L1 höher ist als das Verhältnis von W2 zu L2; und
wobei das Verhältnis von L2 zu L1 von 1,2 bis 2,5 beträgt.

2. Einwegabsorptionshöschen nach Anspruch 1, wobei der Halspunkt (220) des ersten Kanals (126) zum hinteren Ende (216) von 5 % bis 30 % weg von der Mitte (214) des ersten Kanals (126) beabstandet ist, basierend auf der Gesamtlänge des ersten Kanals (126), wie entlang einer geraden Linie vom vorderen Ende (215) zum hinteren Ende (216) des ersten Kanals (126) gemessen; und wobei der Halspunkt (220) des zweiten Kanals (226) zum hinteren Ende (216) von 5 % bis 30 % weg von der Mitte (214) des zweiten Kanals (226) beabstandet ist, basierend auf der Gesamtlänge des zweiten Kanals (226), wie entlang einer geraden Linie vom vorderen Ende (215) zum hinteren Ende (216) des zweiten Kanals (226) gemessen.

3. Einwegabsorptionshöschen nach einem der vorstehenden Ansprüche, wobei der erste Kanal (126) und der zweite Kanal (226) an jeder anderen Stelle nicht näher an der Längsachse (y) liegen als an ihrem Halspunkt (220).

4. Einwegabsorptionshöschen nach einem der vorstehenden Ansprüche, wobei der erste Kanal (126) einem ersten gekrümmten Pfad (221) mit nur einer Kurve zwischen dem Halspunkt (220) und dem hinteren Ende (216) des ersten Kanals (126) folgt und der erste Kanal (126) einem zweiten gekrümmten Pfad (222) mit nur einer Kurve zwischen dem Halspunkt (220) und dem vorderen Ende (215) des ersten Kanals (126) folgt, wobei der erste gekrümmte Pfad (221) des ersten Kanals (126) eine steilere Krümmung im Vergleich zu dem zweiten gekrümmten Pfad (222) des ersten Kanals (126) aufweist; und
wobei der zweite Kanal (226) einem ersten gekrümmten Pfad (221) mit nur einer Kurve zwischen dem Halspunkt (220) und dem hinteren Ende (216) des zweiten Kanals (226) folgt und der zweite Kanal (226) einem zweiten gekrümmten Pfad (222) mit nur einer Kurve zwischen dem Halspunkt (220) und dem vorderen Ende (215) des zweiten Kanals (226) folgt, wobei der erste gekrümmte Pfad (221) des zweiten Kanals (226) eine steilere Krümmung im Vergleich zu dem zweiten gekrümmten Pfad (222) des zweiten Kanals (226) aufweist.

5. Einwegabsorptionshöschen nach einem der vorstehenden Ansprüche, wobei die Absorptionsschicht (117) weniger als 10 Gew.-% Absorptionsmaterial, basierend auf der Gesamtmenge von Absorptionsmaterial in der Absorptionsschicht, in einer Fläche, die sich vom hinteren Rand (219) der Absorptionsschicht zur Querachse (x) erstreckt und 20 % der Längsabmessung der Absorptionsschicht (117), basierend auf der Gesamtlänge der Absorptionsschicht (117), wie vom vorderen Rand (119) zum hinteren Rand (219) der Absorptionsschicht (117) entlang der Längsachse (y) gemessen, überspannt, umfasst.

6. Höschen nach einem der vorstehenden Ansprüche, wobei der erste und der zweite Kanal (126,226) an ihren vorderen Enden (215) um einen dritten Abstand L3 voneinander beabstandet sind, wobei die Absorptionsschicht (117) eine dritte Querbreite W3 aufweist, die sich vom ersten Längsseitenrand (118) zum zweiten Längsseitenrand (218) über die vorderen Enden (215) des ersten und des zweiten Kanals (126, 226) erstreckt,
wobei das Verhältnis von W3 zu L3 von 1,5 bis 2,8 beträgt,
wobei das Verhältnis von W1 zu L1 höher ist als das Verhältnis von W3 zu L3 und
wobei das Verhältnis von L3 zu L1 von 1,2 bis 2,5 oder von 1,4 bis 2,2 oder von 1,5 bis 2,0 beträgt.

7. Höschen nach Anspruch 5, wobei sich L2 und L3 basierend auf dem längeren Abstand nicht um mehr als 30 % voneinander unterscheiden.

8. Höschen nach einem der vorstehenden Ansprüche, wobei das Verhältnis von W1 zu L1 von 2,5 bis 4,5, vorzugsweise von 2,8 bis 4,0 und noch mehr bevorzugt von 3,0 bis 3,8 beträgt.

9. Höschen nach einem der vorstehenden Ansprüche, wobei der elastifizierte vordere Bund (22) einen ersten quer verlaufenden ersten unteren Rand (18) aufweist, der zwischen dem vorderen Taillenrand (34) und der Querachse (x) angeordnet ist, und der elastifizierte hintere Bund (23) einen quer verlaufenden zweiten unteren Rand (19) aufweist, der zwischen dem hinteren Taillenrand (35) und der Querachse (x) angeordnet ist, und die zentrale Grundeinheit (30) mit der körperseitigen Oberfläche des vorderen Bundes (22) angrenzend an ihren Grundeinheit-Vorderrand (135) verbunden ist und mit der körperseitigen Oberfläche des hinteren Bundes (23) angrenzend an ihren Grundeinheit-Hinterrand (136) verbunden ist.

10. Höschen nach Anspruch 9, wobei das Höschen eine Gesamtlänge L4 aufweist, wie vom vorderen Taillenrand (34) zum hinteren Taillenrand (35) entlang der Längsachse (y) gemessen, wobei das Höschen ferner eine Schrittbereichlänge L5 aufweist, wie vom ersten unteren Rand (18) zum zweiten unteren Rand (19) gemessen, wobei das Verhältnis der Gesamtlänge L4 zur Schrittbereichlänge L5 von 1,9 bis 2,6 beträgt; oder von 2,0 bis 2,5.

11. Höschen nach Anspruch 9 oder 10, wobei das hintere Ende (216) des ersten und des zweiten Kanals (126, 226) vom zweiten unteren Rand (19) um von 14 mm bis 25 mm beabstandet ist.

12. Höschen nach einem der vorstehenden Ansprüche, wobei die erste Querbreite W1 der Absorptionsschicht (117), die sich vom ersten Längsseitenrand (118) zum zweiten Längsseitenrand (218) über den Halspunkt (220) des ersten und zweiten Kanals (126, 226) erstreckt, von 50 mm bis 120 mm oder von 60 mm bis 115 mm beträgt.

13. Höschen nach einem der vorstehenden Ansprüche, wobei der erste und der zweite Kanal (126, 226) eine Breite von 5 bis 12 mm oder von 5 mm bis 10 mm aufweisen.

14. Höschen nach einem der vorstehenden Ansprüche, wobei der erste und der zweite Kanal (126, 226) im Wesentlichen Spiegelbilder voneinander im Wesentlichen ohne Versatz zueinander entlang der Längsachse (y) sind.

15. Höschen nach einem der vorstehenden Ansprüche, wobei die Länge jedes des ersten und des zweiten Kanals (126, 226), wie entlang der Längsachse (y) gemessen, von 40 % bis 70 % oder von 45 % bis 65 % oder von 50 % bis 60 % der Gesamtlänge der Absorptionsschicht (117) beträgt, wobei die Gesamtlänge der Absorptionsschicht (117) entlang einer geraden Linie entlang der Längsachse (y) vom vorderen Rand (119) zum hinteren Rand (219) der Absorptionsschicht (117) gemessen wird.

16. Höschen nach einem der vorstehenden Ansprüche, wobei jeder Bund (22, 23) eine Innenschicht (25a), eine Außenschicht (25b) und eine Vielzahl von elastischen Elementen (26) aufweist, die zwischen der Innenschicht und der Außenschicht angeordnet ist.

17. Höschen nach einem der vorstehenden Ansprüche, wobei der Absorptionskern (115) eine erste Substratschicht (116), wie eine Vliesbahn, in Richtung der Unterschicht (31) und eine zweite Substratschicht (116'), wie eine Vliesbahn, in Richtung der Oberschicht (33), und Absorptionsmaterial (150), das zwischen der ersten und der zweiten Substratschicht (116, 116') bereitgestellt ist, umfasst, wobei das Absorptionsmaterial (150) die Absorptionsschicht (117) bildet.

18. Höschen nach einem der Ansprüche 17, wobei der erste und der zweite Kanal (126, 226) im Absorptionskern im Wesentlichen frei von Absorptionsmaterial (150) sind.

19. Höschen nach einem der Ansprüche 17 oder 18, wobei das Absorptionsmaterial (150) zu mindestens 90 % Superabsorberpolymerteilchen umfasst, bezogen auf das Gesamtgewicht des Absorptionsmaterials (150), das zwischen der ersten und der zweiten Substratschicht (116, 116') bereitgestellt ist.

20. Höschen nach einem der Ansprüche 1 bis 18, wobei die Absorptionsschicht Superabsorberpolymerteilchen und Cellulosefasern umfasst.

21. Höschen nach einem der vorstehenden Ansprüche, wobei sich die Absorptionsschicht (117) mit dem vorderen und dem hinteren Bund (22, 23) überlappt.

22. Höschen nach einem der vorstehenden Ansprüche, wobei sich das hintere Ende (216) des ersten und des zweiten Kanals (126, 226) nicht mit dem hinteren Gurt (23) überlappt und das hintere Ende (216) des ersten und des zweiten Kanals (126, 226) vom zweiten unteren Rand (19) um 14 mm bis 25 mm beabstandet ist.

23. Höschen nach einem der vorstehenden Ansprüche, wobei die zentrale Grundeinheit (30) ferner ein Aufnahme-/Verteilungssystem (131) umfasst, das zwischen dem Absorptionskern (115) und der Oberschicht (33) angeordnet ist, wobei das Aufnahme-/Verteilungssystem (131) eine oder mehrere Schichten (132, 133) umfasst, wobei mindestens eine der Schichten (133) längs verlaufende erste und zweite Öffnungen (141, 142) aufweist, wobei jede der ersten und der zweiten Öffnung (141, 142) ein vorderes Öffnungsende und ein hinteres Öffnungsende aufweist, wobei die erste und die zweite Öffnung (141, 142) deckungsgleich mit dem ersten und dem zweiten Kanal (126, 226) sind, jedoch kürzer als der erste und der zweite Kanal (126, 226) sind, sodass ihre vorderen Öffnungsenden in Längsrichtung vom vorderen Ende (215) des ersten bzw. zweiten Kanals (126, 226) versetzt sind und ihre hinteren Öffnungsenden in Längsrichtung von den hinteren Enden (216) des ersten bzw. zweiten Kanals (126, 226) versetzt sind.

24. Höschen nach Anspruch 23, wobei das Aufnahme-/Verteilungssystem (131) zwei Schichten aufweist, eine obere Schicht (132), die angrenzend an die Oberschicht (33) bereitgestellt ist, und eine untere Schicht (133), die angrenzend an den Absorptionskern (115) bereitgestellt ist, wobei die längs verlaufende erste und zweite Öffnung (141, 142), die in der unteren Schicht (133) bereitgestellt sind, und die obere Schicht (132) frei von Öffnungen ist, wobei die obere Schicht (132) durch die erste und die zweite Öffnung (141, 142) der unteren Schicht (133) direkt am Absorptionskern (115) befestigt ist.

## Revendications

1. Culotte absorbante jetable, ayant un axe longitudinal (y), un axe latéral (x), une région de taille avant, une région de taille arrière et une région d'entrejambe entre les régions de taille avant et arrière, la culotte comprenant :
un châssis central (30) s'étend de la région de taille avant à travers la région d'entrejambe à la région de taille arrière et comprend une feuille de dessus (33), une feuille de fond (31) et une âme absorbante (115) placée entre la feuille de dessus (33) et la feuille de fond (31), le châssis central (30) ayant un bord avant de châssis s'étendant latéralement (135), un bord arrière de châssis s'étendant latéralement (136) et des premier et second bords latéraux de châssis s'étendant longitudinalement (137, 138) ; et
une ceinture avant élastifiée (22) prévue dans la région de taille avant et une ceinture arrière élastifiée (23) prévue dans la région de taille arrière, la ceinture avant et arrière (22, 23) ayant chacune une surface faisant face au corps et une surface faisant face au vêtement, la ceinture avant (22) ayant un bord de taille avant s'étendant transversalement (34), la ceinture arrière (23) ayant un bord de taille arrière s'étendant transversalement (35), la ceinture avant et arrière (22, 23) ayant chacune un premier et un second bord latéral s'étendant longitudinalement (230, 231), avec le premier bord latéral (230) de la ceinture avant (22) étant attaché au premier bord latéral (230) de la ceinture arrière (23) et le second bord latéral (231) de la ceinture avant (22) étant attaché au second bord latéral (231) de la ceinture arrière (23) au niveau de coutures latérales (24) pour former une ouverture de taille et deux ouvertures de jambes (15) ; et
l'âme absorbante (115) comprenant une couche absorbante (117), la couche absorbante (117) ayant un bord avant s'étendant latéralement (119), un bord arrière s'étendant latéralement (219) et des premier et second bords latéraux s'étendant longitudinalement (118, 218), la couche absorbante (117) comprenant un premier et un second canal allongé orienté longitudinalement (126, 226) formés en son sein ; et
le premier canal (126) étant prévu entre l'axe longitudinal (y) et le premier bord latéral (118) de la couche absorbante (117), le second canal (226) étant prévu entre l'axe longitudinal (y) et le second bord latéral (218) de la couche absorbante (117) ; et
le premier et le second canal (126, 226) ayant chacun une extrémité avant de canal (215) vers le bord avant de la couche absorbante (119), une extrémité arrière de canal (216) vers le bord arrière de la couche absorbante (219) et un centre (214) qui est équidistant des extrémités avant et arrière du canal (215, 216) à travers l'axe longitudinal (y), chacun du premier et du second canal (126, 226) étant courbé
- de telle sorte que le premier canal (126) est plus proche de l'axe longitudinal (y) au niveau d'un point de rétrécissement que l'extrémité avant et arrière (216) du premier canal (126), et
- de telle sorte que le second canal (226) est plus proche de l'axe longitudinal (y) au niveau d'un point de rétrécissement (220) que l'extrémité avant et arrière (216) du second canal (226),
le premier et le second canal (126, 226) étant espacés l'un de l'autre au niveau de leur point de rétrécissement (220) d'une première distance L1, dans laquelle la distance L1 va de 30 mm à 50 mm
la couche absorbante (117) ayant une première largeur transversale W1 s'étendant du premier bord latéral s'étendant longitudinalement (118) au second bord latéral s'étendant longitudinalement (218) à travers le point de rétrécissement du premier et du second canal ;
le premier et le second canal (126, 226) étant espacés l'un de l'autre au niveau de leurs extrémités arrière (216) d'une deuxième distance L2, la couche absorbante (117) ayant une deuxième largeur transversale W2 s'étendant du premier bord latéral s'étendant longitudinalement (118) au second bord latéral s'étendant longitudinalement (218) à travers les extrémités arrière (216) du premier et du second canal (126, 226),
dans laquelle le rapport de W2 à L2 va de 1,5 à 2,8 ;
dans laquelle le rapport de W1 à L1 est supérieur au rapport de W2 à L2 ; et
dans laquelle le rapport de L2 à L1 va de 1,2 à 2,5.

2. Culotte absorbante jetable selon la revendication 1, dans laquelle le point de rétrécissement (220) du premier canal (126) est espacé de 5 % à 30 % à l'écart du centre (214) du premier canal (126) vers l'extrémité arrière (216), en fonction de la longueur totale du premier canal (126), telle que mesurée le long d'une ligne droite de l'extrémité avant (215) à l'extrémité arrière (216) du premier canal (126) ; et dans laquelle le point de rétrécissement (220) du second canal (226) est espacé de 5 % à 30 % à l'écart du centre (214) du second canal (226) vers l'extrémité arrière (216), en fonction de la longueur totale du second canal (226), telle que mesurée le long d'une ligne droite de l'extrémité avant (215) à l'extrémité arrière (216) du second canal (226).

3. Culotte absorbante jetable selon l'une quelconque des revendications précédentes, dans laquelle le premier canal (126) et le second canal (226) ne sont pas plus proches de l'axe longitudinal (y) au niveau de l'un quelconque autre emplacement qu'au niveau de leur point de rétrécissement (220).

4. Culotte absorbante jetable selon l'une quelconque des revendications précédentes, dans laquelle le premier canal (126) suit un premier trajet courbé (221) avec une seule courbe entre le point de rétrécissement (220) et l'extrémité arrière (216) du premier canal (126), et le premier canal (126) suit un second trajet courbé (222) avec une seule courbe entre le point de rétrécissement (220) et l'extrémité avant (215) du premier canal (126), dans laquelle le premier trajet courbé (221) du premier canal (126) a une courbure plus abrupte par comparaison avec le second trajet courbé (222) du premier canal (126) ; et
dans laquelle le second canal (226) suit un premier trajet courbé (221) avec une seule courbe entre le point de rétrécissement (220) et l'extrémité arrière (216) du second canal (226), et le second canal (226) suit un second trajet courbé (222) avec une seule courbe entre le point de rétrécissement (220) et l'extrémité avant (215) du second canal (226), dans laquelle le premier trajet courbé (221) du second canal (226) a une courbure plus abrupte par comparaison avec le second trajet courbé (222) du second canal (226).

5. Culotte absorbante jetable selon l'une quelconque des revendications précédentes, dans laquelle la couche absorbante (117) comprend moins de 10 % en poids de matériau absorbant, en fonction de la quantité totale de matériau absorbant dans la couche absorbante, dans une zone s'étendant du bord arrière (219) de la couche absorbante vers l'axe latéral (x) et couvrant 20 % de la dimension longitudinale de la couche absorbante (117), en fonction de la longueur totale de la couche absorbante (117) telle que mesurée du bord avant (119) au bord arrière (219) de la couche absorbante (117) le long de l'axe longitudinal (y).

6. Culotte selon l'une quelconque des revendications précédentes, dans laquelle le premier et le second canal (126, 226) sont espacés l'un de l'autre au niveau de leurs extrémités avant (215) d'une troisième distance L3, la couche absorbante (117) ayant une troisième largeur transversale W3 s'étendant du premier bord latéral s'étendant longitudinalement (118) au second bord latéral s'étendant longitudinalement (218) à travers les extrémités avant (215) du premier et du second canal (126, 226),
dans laquelle le rapport de W3 à L3 va de 1,5 à 2,8,
dans laquelle le rapport de W1 à L1 est supérieur au rapport de W3 à L3, et
dans laquelle le rapport de L3 à L1 va de 1,2 à 2,5, ou de 1,4 à 2,2, ou de 1,5 à 2,0.

7. Culotte selon la revendication 5, dans laquelle L2 et L3 ne diffèrent pas l'une de l'autre de plus de 30 % en fonction de la distance plus longue.

8. Culotte selon l'une quelconque des revendications précédentes, dans laquelle le rapport de W1 à L1 va de 2,5 à 4,5, de préférence de 2,8 à 4,0, et encore plus préférablement de 3,0 à 3,8.

9. Culotte selon l'une quelconque des revendications précédentes, dans laquelle la ceinture avant élastifiée (22) a un premier bord inférieur s'étendant transversalement (18) disposé entre le bord de taille avant (34) et l'axe latéral (x), et la ceinture arrière élastifiée (23) a un second bord inférieur s'étendant transversalement (19), disposé entre le bord de taille arrière (35) et l'axe latéral (x), et le châssis central (30) est attaché à la surface faisant face au corps de la ceinture avant (22) adjacente à son bord avant de châssis (135) et attaché à la surface faisant face au corps de la ceinture arrière (23) adjacente à son bord arrière de châssis (136).

10. Culotte selon la revendication 9, dans laquelle la culotte a une longueur globale L4 telle que mesurée du bord de taille avant (34) au bord de taille arrière (35) le long de l'axe longitudinal (y), la culotte ayant en outre une longueur de région d'entrejambe L5 telle que mesurée du premier bord inférieur (18) au second bord inférieur (19), dans laquelle le rapport de la longueur globale L4 à la longueur de région d'entrejambe L5 va de 1,9 à 2,6 ; ou de 2,0 à 2,5.

11. Culotte selon la revendication 9 ou 10, dans laquelle l'extrémité arrière (216) du premier et du second canal (126, 226) est espacée du second bord inférieur (19) de 14 mm à 25 mm.

12. Culotte selon l'une quelconque des revendications précédentes, dans laquelle la première largeur transversale W1 de la couche absorbante (117) s'étendant du premier bord latéral s'étendant longitudinalement (118) au second bord latéral s'étendant longitudinalement (218) à travers le point de rétrécissement (220) du premier et du second canal (126, 226) va de 50 mm à 120 mm, ou de 60 mm à 115 mm.

13. Culotte selon l'une quelconque des revendications précédentes, dans laquelle les premier et second canaux (126, 226) ont une largeur allant de 5 à 12 mm, ou de 5 mm à 10 mm.

14. Culotte selon l'une quelconque des revendications précédentes, dans laquelle le premier et le second canal (126, 226) sont des images sensiblement miroir l'un de l'autre avec sensiblement pas de décalage l'un par rapport à l'autre le long de l'axe longitudinal (y).

15. Culotte selon l'une quelconque des revendications précédentes, dans laquelle la longueur de chacun du premier et du second canal (126, 226) telle que mesurée le long de l'axe longitudinal (y) va de 40 % à 70 %, ou de 45 % à 65 %, ou de 50 % à 60 % de la longueur totale de la couche absorbante (117), la longueur totale de la couche absorbante (117) étant mesurée le long d'une ligne droite le long de l'axe longitudinal (y) du bord avant (119) au bord arrière (219) de la couche absorbante (117).

16. Culotte selon l'une quelconque des revendications précédentes, dans laquelle chaque ceinture (22, 23) a une couche interne (25a), une couche externe (25b) et une pluralité d'éléments élastiques (26) disposés entre la couche interne et la couche externe.

17. Culotte selon l'une quelconque des revendications précédentes, dans laquelle l'âme absorbante (115) comprend une première couche de substrat (116), telle qu'un voile non tissé, vers la feuille de fond (31) et une seconde couche de substrat (116'), telle qu'un voile non tissé, vers la feuille de dessus (33), et un matériau absorbant (150) prévu entre une première et une seconde couche de substrat (116, 116'), le matériau absorbant (150) formant la couche absorbante (117).

18. Culotte selon l'une quelconque de la revendication 17, dans laquelle les premier et second canaux (126, 226) dans l'âme absorbante sont sensiblement dépourvus de matériau absorbant (150).

19. Culotte selon l'une quelconque des revendications 17 ou 18, dans laquelle le matériau absorbant (150) comprend au moins 90 % de particules de polymère superabsorbant en poids total du matériau absorbant (150) prévu entre la première et la seconde couche de substrat (116, 116').

20. Culotte selon l'une quelconque des revendications 1 à 18, dans laquelle la couche absorbante comprend des particules de polymère superabsorbant et des fibres de cellulose.

21. Culotte selon l'une quelconque des revendications précédentes, dans laquelle la couche absorbante (117) chevauche la ceinture avant et arrière (22, 23).

22. Culotte selon l'une quelconque des revendications précédentes, dans laquelle l'extrémité arrière (216) du premier et du second canal (126, 226) ne chevauche pas la ceinture arrière (23) et l'extrémité arrière (216) du premier et du second canal (126, 226) est espacée à l'écart du second bord inférieur (19) de 14 mm à 25 mm.

23. Culotte selon l'une quelconque des revendications précédentes, dans laquelle le châssis central (30) comprend en outre un système d'acquisition/distribution (131) intercalé entre l'âme absorbante (115) et la feuille de dessus (33), le système d'acquisition/distribution (131) comprenant une ou plusieurs couches (132, 133), au moins l'une des couches (133) ayant des première et seconde ouvertures s'étendant longitudinalement (141, 142), chacune de la première et la seconde ouverture (141, 142) ayant une extrémité d'ouverture avant et une extrémité d'ouverture arrière, les première et seconde ouvertures (141, 142) étant congruentes avec les premier et second canaux (126, 226) mais étant plus courtes que les premier et second canaux (126, 226) de telle sorte que leurs extrémités d'ouverture avant sont longitudinalement décalées de l'extrémité avant (215) du premier et du second canal (126, 226), respectivement, et leurs extrémités d'ouverture arrière sont longitudinalement décalées des extrémités arrière (216) du premier et du second canal (126, 226), respectivement.

24. Culotte selon la revendication 23, dans laquelle le système d'acquisition/distribution (131) a deux couches, une couche supérieure (132) prévue adjacente à la feuille de dessus (33) et une couche inférieure (133) prévue adjacente à l'âme absorbante (115), dans laquelle les première et seconde ouvertures s'étendant longitudinalement (141, 142) sont prévues dans la couche inférieure (133) et la couche supérieure (132) est dépourvue d'ouvertures, la couche supérieure (132) étant directement fixée à l'âme absorbante (115) à travers les première et seconde ouvertures (141, 142) de la couche inférieure (133).
